# EUROPEAN PATENT SPECIFICATION

(11) **EP 2 406 266 B1**
(45) Date of publication and mention of the grant of the patent: **25.12.2013**
(21) Application number: 10750950.7
(22) Date of filing: 10.03.2010
(51) Int. Cl.: C07D 498/06, A61K 31/5365, A61P 1/00, A61P 3/10, A61P 19/00, A61P 25/08, A61P 25/24, A61P 25/28, A61P 29/00, A61P 35/00

(54) **7-CYCLOALKYLAMINOQUINOLONES AS GSK-3 INHIBITORS**
7-CYCLOALKYLAMINOCHINOLONE ALS GSK-3-HEMMER
7-CYCLOALKYLAMINOQUINOLONES COMME INHIBITEURS DE LA GSK-3

(30) Priority: 11.03.2009 US 159409 P
(43) Date of publication of application: 18.01.2012
(73) Proprietor: Kyorin Pharmaceutical Co., Ltd., Tokyo 101-8311 (JP)
(72) Inventor: OKADA, Kyoko, Chiyoda-ku Tokyo 1018311 (JP); SATO, Taro, Shimotsuga-gun Tochigi 329-0114 (JP); KOHNO, Yasushi, Shimotsuga-gun Tochigi 329-0114 (JP); NOMURA, Masahiro, Shimotsuga-gun Tochigi 329-0114 (JP)
(74) Representative: Savic, Bojan
(86) International application number: PCT/JP2010/054479
(87) International publication number: WO 2010/104205

(56) References cited:
- EP-A1- 0 265 230
- WO-A2-2007/106537
- WO-A2-2009/035634

## Description

Compounds, compositions and methods for treating GSK-3 mediated diseases are provided. The compounds provided herein are aminoquinolones that are GSK-3 inhibitors.

### BACKGROUND

Glycogen synthase kinase-3 (GSK-3) is a serine/threonine protein kinase having α and β isoforms that are each encoded by distinct genes [Coghlan et al., Chemistry & Biology, 7, 793-803 (2000); and Kim and Kimmel, Curr. Opinion Genetics Dev., 10, 508-514 (2000)]. GSK-3 has been implicated in various diseases including diabetes, Alzheimer's disease, CNS disorders such as manic depressive disorder and neurodegenerative diseases, and cardiomyocete hypertrophy [see, *e.g*., WO 99/65897; WO 00/38675; and Haq et al., J. Cell Biol. (2000) 151, 117]. These diseases may be caused by, or may result in, the abnormal operation of certain cell signaling pathways in which GSK-3 plays a role.

GSK-3 has been found to phosphorylate and modulate the activity of a number of regulatory proteins. These include glycogen synthase, which is the rate-limiting enzyme required for glycogen synthesis, the microtubule-associated protein Tau, the gene transcription factor β-catenin, the translation initiation factor e1F-2B, as well as ATP citrate lyase, axin, heat shock factor-1, c-Jun, c-myc, c-myb, CREB, and CEPB α. These diverse targets implicate GSK-3 in many aspects of cellular metabolism, proliferation, differentiation and development.

Small molecule inhibitors of GSK-3 have recently been reported [WO 99/65897 (Chiron) and WO 00/38675 (SmithKline Beecham)], however, there is a continued need to find more effective therapeutic agents to treat GSK-3 mediated diseases.

### SUMMARY

1. Provided herein are compounds represented by the Formula (I): 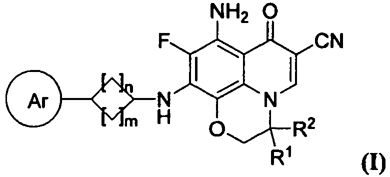 or a pharmaceutically acceptable salt thereof, wherein
R¹ is lower alkyl;
R² is hydrogen or lower alkyl;
m is 1, 2 or 3;
n is 1 or 2;
Ar is aryl or heteroaryl which is optionally substituted with one to three substituents, each independently selected from Q groups;
wherein Q is halo, hydroxy, cyano, nitro, oxo, thioxo, hydroxycarbonyl, alkyl, haloalkyl, alkenyl, alkynyl, cycloalkyl, heterocyclyl, aryl, heteroaryl, aralkyl, heteroaralkyl, alkoxy, haloalkoxy, cycloalkoxy, heterocyclyloxy, aryloxy, heteroaryloxy, aralkyloxy, heretoaralkyloxy, alkylcarbonyl, arylcarbonyl, heteroarylcarbonyl, alkoxycarbonyl, aryloxycarbonyl, aralkyloxycarbonyl, unsubstituted or substituted aminocarbonyl, alkylcarbonyloxy, arylcarbonyloxy, aralkylcarbonyloxy, alkoxycarbonyloxy, aryloxycarbonyloxy, aralkyloxycarbonyloxy, unsubstituted or substituted aminocarbonyloxy, unsubstituted or substituted amino, alkylthio, cycloalkylthio, arylthio, heteroarylthio, aralkylthio, heteroaralkylthio, alkylsulfinyl, cycloalkylsulfinyl, arylsulfinyl, heteroarylsulfinyl, aralkylsulfinyl, heteroaralkylsulfinyl, alkylsulfonyl, cycloalkylsulfonyl, arylsulfonyl, heteroarylsulfonyl, aralkylsulfonyl, heteroaralkylsulfonyl, alkoxysulfonyl, aryloxysulfonyl, unsubstituted or substituted aminosulfonyl, or hydroxysulfonyl.

Also provided herein are pharmaceutical compositions containing a compound of Formula (I) and a pharmaceutically acceptable carrier.

Also described herein are methods for treating, preventing, or ameliorating one or more symptoms of GSK-3 mediated diseases by administering the compounds and compositions provided herein.

### DETAILED DESCRIPTION OF EMBODIMENTS

### A. Definitions

Unless defined otherwise, all technical and scientific terms used herein have the same meaning as is commonly understood by one of ordinary skill in the art. All patents, applications, published applications and other publications are incorporated by reference in their entirety. In the event that there are a plurality of definitions for a term herein, those in this section prevail unless stated otherwise.

As used herein, the nomenclature alkyl, alkoxy, carbonyl, etc. is used as is generally understood by those of skill in this art.

As used herein, alkyl carbon chains, if not specified, contain from 1 to 20 carbons, 1 to 16 carbons or 1 to 6 carbons and are straight or branched. In certain embodiments, alkyl carbon chains contain from 1 to 6 carbons. Exemplary alkyl groups herein include, but are not limited to, methyl, ethyl, propyl, isopropyl, isobutyl, n-butyl, sec-butyl, tert-butyl, isopentyl, neopentyl, tert-pentyl, isohexyl. As used herein, lower alkyl refers to carbon chains having from about 1 carbons up to about 6 carbons.

As used herein, alkenyl carbon chains, if not specified, contain from 2 to 20 carbons, 2 to 16 carbons or 2 to 6 carbons and are straight or branched. In certain embodiments, alkenyl carbon chains contain from 2 to 6 carbons. Alkenyl carbon chains of from 2 to 20 carbons, in certain embodiments, contain 1 to 8 double bonds, and the alkenyl carbon chains of 2 to 16 carbons, in certain embodiments, contain 1 to 5 double bonds. The alkenyl carbon chains of 2 to 6 carbons, in certain embodiments, contain 1 to 2 double bonds. Exemplary alkenyl groups herein include, but are not limited to, vinyl, 1-propenyl, 2-propenyl, isopropenyl, 1-butenyl, 2-butenyl, 3-butenyl, 1,3-butadienyl. As used herein, lower alkenyl refer to carbon chains having from about 2 carbons up to about 6 carbons.

As used herein, alkynyl carbon chains, if not specified, contain from 2 to 20 carbons, 2 to 16 carbons or 2 to 6 carbons and are straight or branched. In certain embodiments, alkynyl carbon chains contain from 2 to 6 carbons. Alkynyl carbon chains of from 2 to 20 carbons, in certain embodiments, contain 1 to 8 triple bonds, and the alkynyl carbon chains of 2 to 16 carbons, in certain embodiments, contain 1 to 5 triple bonds. Alkynyl carbon chains of from 2 to 6 carbons, in certain embodiments, contain 1 to 2 triple bonds. Exemplary alkynyl groups herein include, but are not limited to, ethynyl, 1-propynyl and 2-propynyl. As used herein, lower alkynyl refer to carbon chains having from about 2 carbons up to about 6 carbons.

As used herein, "alkoxy" contain from 1 to 20 carbons, 1 to 16 carbons or 1 to 6 carbons and are straight or branched. In certain embodiments, alkoxy carbon chains contain from 1 to 6 carbons. Exemplary alkoxy groups herein include, but are not limited to, methoxy, ethoxy, propoxy, isopropoxy, isobutoxy, n-butoxy, sec-butoxy, tert-butoxy, isopentoxy, neopentoxy, tert-pentoxy and isohexyloxy.

As used herein, "aralkyl" refers to an alkyl group in which one of the hydrogen atoms of the alkyl is replaced by an aryl.

As used herein, "halo", "halogen" or "halide" refers to F, Cl, Br or I.

As used herein, "aryl" refers to aromatic monocyclic or multicyclic groups containing from 6 to 19 carbon atoms. Aryl groups include, but are not limited to groups such as fluorenyl, substituted fluorenyl, phenyl, substituted phenyl, naphthyl and substituted naphthyl.

As used herein, "cycloalkyl" refers to a saturated mono- or multicyclic ring system, in certain embodiments of 3 to 10 carbon atoms, in other embodiments of 3 to 6 carbon atoms; cycloalkenyl and cycloalkynyl refer to mono- or multicyclic ring systems that respectively include at least one double bond and at least one triple bond. Cycloalkenyl and cycloalkynyl groups may, in certain embodiments, contain 3 to 10 carbon atoms, with cycloalkenyl groups, in further embodiments, containing 4 to 7 carbon atoms and cycloalkynyl groups, in further embodiments, containing 8 to 10 carbon atoms. The ring systems of the cycloalkyl, cycloalkenyl and cycloalkynyl groups may be composed of one ring or two or more rings which may be joined together in a fused, bridged or spiro-connected fashion.

As used herein, "heterocyclyl" refers to a monocyclic or multicyclic non-aromatic ring system, in one embodiment of 3 to 10 members, in another embodiment of 4 to 7 members, in a further embodiment of 5 to 6 members, where one or more, in certain embodiments, 1 to 3, of the atoms in the ring system is a heteroatom, that is, an element other than carbon, including but not limited to, nitrogen, oxygen or sulfur. In embodiments where the heteroatom(s) is(are) nitrogen, the nitrogen is optionally substituted with alkyl, alkenyl, alkynyl, aryl, heteroaryl, aralkyl, heteroaralkyl, cycloalkyl, heterocyclyl, cycloalkylalkyl, heterocyclylalkyl, acyl, aminocarbonyl, alkoxycarbonyl, guanidino, or the nitrogen may be quatemized to form an ammonium group where the substituents are selected as above.

As used herein, "heteroaryl" refers to a monocyclic or multicyclic aromatic ring system, in certain embodiments, of about 5 to about 15 members where one or more, in one embodiment 1 to 3, of the atoms in the ring system is a heteroatom, that is, an element other than carbon, including but not limited to, nitrogen, oxygen or sulfur. The heteroaryl group may be optionally fused to a benzene ring. Heteroaryl groups include, but are not limited to, furyl, imidazolyl, pyrimidinyl, tetrazolyl, thienyl, pyridyl, pyrrolyl, N-methylpyrrolyl, quinolinyl and isoquinolinyl.

As used herein, "fused heterocyclylaryl" refers to aryl which was fused with a heterocyclyl group. In one embodiment, fused heterocyclylaryls are those wherein heterocyclyl contains about 5 to about 6 ring atoms and the aryl thereof is phenyl. A fused heterocyclylaryl may be bonded through any atom of the ring system. Representative fused heterocyclylaryl groups include 1,3-benzodioxolan-4-yl, 1,3-benzodioxolan-5-yl, 1,3-benzodioxolan-6-yl, 1,3-benzodioxolan-7-yl, 4-indolinyl, 5-indolinyl, 6-indolinyl and 7-indolinyl.

As used herein, "fused arylheterocyclyl" refers to fused heterocyclyl which was fused to an aryl group. In one embodiment, fused arylheterocyclyls are those wherein the aryl thereof is phenyl and the heterocyclyl contains about 5 to about 6 ring atoms. A fused arylheterocyclyl may be bonded through any atom of the ring system. Representative fused arylheterocyclyl groups include 1-indolinyl, 2-indolinyl, 3-indolinyl, 1,2,3,4-tetrahydroqunolin-1-yl, 1,2,3,4-tetrahydroqunolin-2-yl, 1,2,3,4-tetrahydroqunolin-3-yl and 1,2,3,4-tetrahydroqunolin-4-yl.

As used herein, "haloalkyl" refers to an alkyl group in which one or more of the hydrogen atoms are replaced by halogen. "Lower haloalkyl" refers to a lower alkyl group in which one or more of the hydrogen atoms are replaced by halogen. Such groups include, but are not limited to, chloromethyl, trifluoromethyl and 1-chloro-2-fluoroethyl.

As used herein, "heteroaralkyl" refers to an alkyl group in which one of the hydrogen atoms are replaced by heteroaryl.

As used herein, "haloalkoxy" refers to RO- in which R is a haloalkyl group.

As used herein, "cycloalkoxy" refers to RO- in which R is a cycloalkyl group.

As used herein, "aryloxy" refers to RO- in which R is an aryl.

As used herein, "heteroaryloxy" refers to RO- in which R is a heteroaryl.

As used herein, "heterocyclyloxy" refers to RO- in which R is a heterocyclyl.

As used herein, "aralkyloxy" refers to RO- in which R is an aralkyl.

As used herein, "heteroaralkyloxy" refers to RO- in which R is a heteroaralkyl.

As used herein, "alkylcarbonyl" refers to RCO- in which R is an alkyl group.

As used herein, "arylcarbonyl" refers to RCO- in which R is an aryl.

As used herein, "heteroarylcarbonyl " refers to RCO- in which R is a heteroaryl.

As used herein, "alkoxycarbonyl" refers to RCO- in which R is an alkoxy group.

As used herein, "aryloxycarbonyl" refers to RCO- in which R is an aryloxy.

As used herein, "aralkyloxycarbonyl" refers to RCO- in which R is an aralkyloxy.

As used herein, "unsubstituted or substituted aminocarbonyl" refers to -C(O)N R' R in which R' and R are independently hydrogen, alkyl aryl, heteroaryl, aralkyl or heteroaralkyl.

As used herein, "alkylcarbonyloxy" refers to -OC(O) R in which R is alkyl.

As used herein, "arylcarbonyloxy" refers to -OC(O) R in which R is aryl.

As used herein, "aralkylcarbonyloxy" refers to -OC(O) R in which R is aralkyl.

As used herein, "alkoxycarbonyloxy" refers to -OC(O)O R in which R is alkyl.

As used herein, "aryloxycarbonyloxy" refers to -OC(O)O R in which R is aryl.

As used herein, "aralkyloxycarbonyloxy" refers to -OC(O)O R in which R is aralkyl.

As used herein, "unsubstituted or substituted aminocarbonyloxy" refers to -OC(O) NR'R in which R' and R are independently hydrogen, alkyl aryl, heteroaryl, aralkyl or heteroaralkyl.

As used herein, "unsubstituted or substituted amino" refers to -NR'R in which R' and R are independently hydrogen, alkyl, aryl, heteroaryl, aralkyl, heteroaralkyl, alkylcarbonyl, arylcarbonyl, heteroarylcarbonyl, alkoxycarbonyl, aryloxycarbonyl, aralkyloxycarbonyl, unsubstituted or substituted aminocarbonyl, alkylsulfonyl, cycloalkylsulfonyl, arylsulfonyl, heteroarylsulfonyl, aralkylsulfonyl, or heteroaralkylsulfonyl.

As used herein, "alkylthio" refers to - SR in which R is alkyl.

As used herein, "cycloalkylthio" refers to - SR in which R is cycloalkyl.

As used herein, "arylthio" refers to - SR in which R is aryl.

As used herein, "heteroarylthio" refers to - SR in which R is heteroaryl.

As used herein, "aralkylthio" refers to - SR in which R is aralkyl.

As used herein, "heteroaralkylthio" refers to - SR in which R is heteroaralkyl.

As used herein, "alkylsulfinyl" refers to - S(O)R in which R is alkyl.

As used herein, "cycloalkylsulfinyl" refers to - S(O)R in which R is cycloalkyl.

As used herein, "arylsulfinyl" refers to - S(O)R in which R is aryl.

As used herein, "heteroarylsulfinyl" refers to - S(O)R in which R is heteroaryl.

As used herein, "aralkylsulfinyl" refers to - S(O)R in which R is aralkyl.

As used herein, "heteroaralkylsulfinyl" refers to - S(O)R in which R is heteroaralkyl.

As used herein, "alkylsulfonyl" refers to - S(O)₂R in which R is alkyl.

As used herein, "cycloalkylsulfonyl" refers to - S(O)₂R in which R is cycloalkyl.

As used herein, "arylsulfonyl" refers to - S(O)₂R in which R is aryl.

As used herein, "heteroarylsulfonyl" refers to - S(O)₂R in which R is heteroaryl.

As used herein, "alkoxysulfonyl" refers to -S(O)₂R in which R is alkoxy.

As used herein, "aryloxysulfonyl" refers to -S(O)₂R in which R is aryloxy.

As used herein, "aralkylsulfonyl" refers to - S(O)₂R in which R is aralkyl.

As used herein, "heteroaralkylsulfonyl" refers to - S(O)₂R in which R is heteroaralkyl.

As used herein, "unsubstituted or substituted aminosulfonyl" refers to - S(O)₂N R'R in which R' and R are independently hydrogen, alkyl aryl, heteroaryl, aralkyl or heteroaralkyl.

As used herein, pharmaceutically acceptable salts include, but are not limited to, amine salts, such as but not limited to N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethylbenzimidazole, diethylamine and other alkylamines, piperazine and tris(hydroxymethyl)aminomethane; alkali metal salts, such as but not limited to lithium, potassium and sodium; alkali earth metal salts, such as but not limited to barium, calcium and magnesium; transition metal salts, such as but not limited to zinc; and inorganic salts, such as but not limited to, sodium hydrogen phosphate and disodium phosphate; and also including, but not limited to, salts of mineral acids, such as but not limited to hydrochlorides and sulfates; and salts of organic acids, such as but not limited to acetates, lactates, malates, tartrates, citrates, ascorbates, succinates, butyrates, valerates, mesylates, and fumarates.

It is to be understood that the compounds provided herein may contain chiral centers. Such chiral centers may be of either the (R) or (S) configuration, or may be a mixture thereof. Thus, the compounds provided herein may be enantiomerically pure, or be stereoisomeric or diastereomeric mixtures.

As used herein, substantially pure means sufficiently homogeneous to appear free of readily detectable impurities as determined by standard methods of analysis, such as thin layer chromatography (TLC), gel electrophoresis, high performance liquid chromatography (HPLC), nuclear magnetic resonance (NMR), and mass spectrometry (MS), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound may, however, be a mixture of stereoisomers. In such instances, further purification might increase the specific activity of the compound. The instant disclosure is meant to include all such possible isomers, as well as, their racemic and optically pure forms. Optically active (+) and (-), (R)- and (S)-, or (D)- and (L)-isomers may be prepared using chiral synthons or chiral reagents, or resolved using conventional techniques, such as reverse phase HPLC. When the compounds described herein contain olefinic double bonds or other centers of geometric asymmetry, and unless specified otherwise, it is intended that the compounds include both E and Z geometric isomers. Likewise, all tautomeric forms are also intended to be included.

As used herein, the IC₅₀ refers to an amount, concentration or dosage of a particular test compound that achieves a 50% inhibition of a maximal response in an assay that measures such response.

As used herein, EC₅₀ refers to a dosage, concentration or amount of a particular test compound that elicits a dose-dependent response at 50% of maximal expression of a particular response that is induced, provoked or potentiated by the particular test compound.

As used herein, treatment means any manner in which one or more of the symptoms of a disease or disorder are ameliorated or otherwise beneficially altered. Treatment also encompasses any pharmaceutical use of the compositions herein, such as use for treating diabetes.

As used herein, amelioration of the symptoms of a particular disorder by administration of a particular compound or pharmaceutical composition refers to any lessening, whether permanent or temporary, lasting or transient that can be attributed to or associated with administration of the composition.

As used herein, the terms "preventing" means guard against the recurrence of the specified disease or disorder in a patient who has already suffered from the disease or disorder, and/or lengthening the time that a patient who has suffered from the disease or disorder remains in remission.

The terms "GSK-3 mediated disease, or "GSK-3 mediated condition", as used herein, mean any disease or other deleterious condition or state in which GSK-3 is known to play a role. Such diseases or conditions include, without limitation, diabetes, conditions associated with diabetes, chronic neurodegenerative conditions including dementias such as Alzheimer's disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis, neurotraumatic diseases such as acute stroke, epilepsy, mood disorders such as depression, schizophrenia and bipolar disorders, rheumatoid arthritis, inflammatory bowel disease, ulceractive colitis, Crohn's disease, sepsis, pancreatic cancer, ovarian cancer and osteoporosis.

### B. Compounds

Provided herein are compounds of Formula (I): 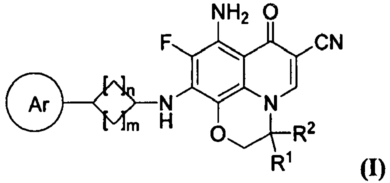 or a pharmaceutically acceptable salt thereof, wherein
R¹ is lower alkyl;
R² is hydrogen or lower alkyl;
m is 1, 2 or 3;
n is 1 or 2;
Ar is aryl or heteroaryl which is optionally substituted with one to three substituents, each independently selected from Q groups;
wherein Q is halo, hydroxy, cyano, nitro, oxo, thioxo, hydroxycarbonyl, alkyl, haloalkyl, alkenyl, alkynyl, cycloalkyl, heterocyclyl, aryl, heteroaryl, aralkyl, heteroaralkyl, alkoxy, haloalkoxy, cycloalkoxy, heterocyclyloxy, aryloxy, heteroaryloxy, aralkyloxy, heretoaralkyloxy, alkylcarbonyl, arylcarbonyl, heteroarylcarbonyl, alkoxycarbonyl, aryloxycarbonyl, aralkyloxycarbonyl, unsubstituted or substituted aminocarbonyl, alkylcarbonyloxy, arylcarbonyloxy, aralkylcarbonyloxy, alkoxycarbonyloxy, aryloxycarbonyloxy, aralkyloxycarbonyloxy, unsubstituted or substituted aminocarbonyloxy, unsubstituted or substituted amino, alkylthio, cycloalkylthio, arylthio, heteroarylthio, aralkylthio, heteroaralkylthio, alkylsulfinyl, cycloalkylsulfinyl, arylsulfinyl, heteroarylsulfinyl, aralkylsulfinyl, heteroaralkylsulfinyl, alkylsulfonyl, cycloalkylsulfonyl, arylsulfonyl, heteroarylsulfonyl, aralkylsulfonyl, heteroaralkylsulfonyl, alkoxysulfonyl, aryloxysulfonyl, unsubstituted or substituted aminosulfonyl, alkoxysulfonyl, aryloxysulfonyl, or hydroxysulfonyl.

In one embodiment, R² is hydrogen.

In another embodiment, Ar is heteroaryl which is optionally substituted with one to three substituents, each independently selected from Q groups..

In another embodiment, m+n is 3 or 4.

In certain embodiments, the compounds are of Formula (Ia): 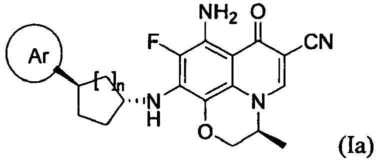 or a pharmaceutically acceptable salt thereof, wherein
n is 1 or 2;
Ar is aryl or heteroaryl which is optionally substituted with one to three substituents, each independently selected from Q groups;
wherein Q is halo, hydroxy, cyano, nitro, oxo, thioxo, hydroxycarbonyl, alkyl, haloalkyl, alkenyl, alkynyl, cycloalkyl, heterocyclyl, aryl, heteroaryl, aralkyl, heteroaralkyl, alkoxy, haloalkoxy, cycloalkoxy, heterocyclyloxy, aryloxy, heteroaryloxy, aralkyloxy, heretoaralkyloxy, alkylcarbonyl, arylcarbonyl, heteroarylcarbonyl, alkoxycarbonyl, aryloxycarbonyl, aralkyloxycarbonyl, unsubstituted or substituted aminocarbonyl, alkylcarbonyloxy, arylcarbonyloxy, aralkylcarbonyloxy, alkoxycarbonyloxy, aryloxycarbonyloxy, aralkyloxycarbonyloxy, unsubstituted or substituted aminocarbonyloxy, unsubstituted or substituted amino, alkylthio, cycloalkylthio, arylthio, heteroarylthio, aralkylthio, heteroaralkylthio, alkylsulfinyl, cycloalkylsulfinyl, arylsulfinyl, heteroarylsulfinyl, aralkylsulfinyl, heteroaralkylsulfinyl, alkylsulfonyl, cycloalkylsulfonyl, arylsulfonyl, heteroarylsulfonyl, aralkylsulfonyl, heteroaralkylsulfonyl, alkoxysulfonyl, aryloxysulfonyl, unsubstituted or substituted aminosulfonyl, or hydroxysulfonyl.

In one embodiment, the compounds are of Formula (Ia), wherein Ar is heteroaryl which is optionally substituted with one to three substituents, each independently selected from Q groups.

In one embodiment, the compounds are of Formula (Ia), wherein Ar is pyridyl, pyramidyl, pyrazolyl or imidazolyl, which is optionally substituted with one to three substituents, each independently selected from Q groups.

In other embodiments, the compounds are selected from:

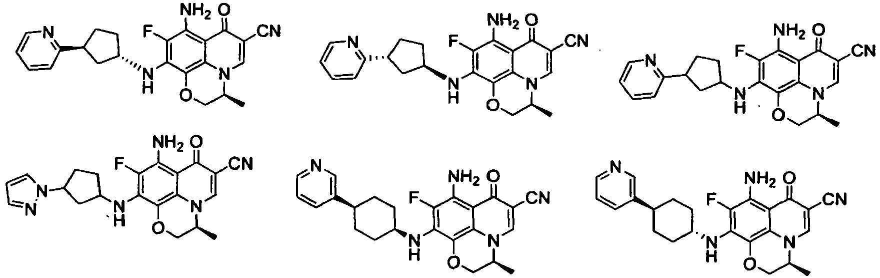

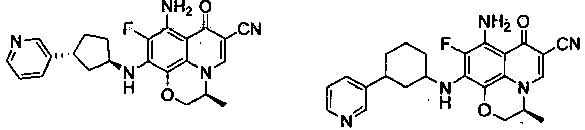

### C. Preparation of the Compounds

The compounds provided herein can be prepared by methods known to one of skill in the art as showed below and following procedures similar to those described in the Examples section herein and routine modifications thereof.
Kyorin Pharmaceutical Co., Ltd., Japanese Unexamined Patent Publication S62-252772,
Ube Industries, Ltd., Japanese Unexamined Patent Publication S63-264439, Bayer AG; Japanese Unexamined Patent Publication H1-268679,
Ihara Chemical Industry Co., Ltd., Japanese Unexamined Patent Publication H10-70584,
Daiichi Seiyaku Co., Ltd., Japanese Unexamined Patent Publication S59-1489, Tokyo Tanabe Co., Ltd., Japanese Unexamined Patent Publication S62-53987,
F. Hoffmann-La Roche Ltd., Japanese Unexamined Patent Publication S63-132891,
H. Koga et al., J. Med. Chem., 1980, 23, 1358,
S. Atarashi et al., Chem. Pharm. Bull., 1987, 35, 1896,
S. Atarashi et al., J. Heterocyclic Chem., 1991, 28, 329,
H. Egawa et al., Chem. Pharm. Bull., 1986, 34, 4098*,*
L. A. Mitscher et al., J. Med. Chem., 1987, 30, 2283,
G. B. Mullen et al., J. Med. Chem., 1988, 31, 1694,
J. S. Kiely et al., J Med. Chem., 1988, 31, 2004,
P. Remuzon et al., J. Med. Chem., 1991, 34, 29,
Warner-Lambert Co., Japanese Unexamined Patent Publication S62-167769,
Shionogi Seiyaku Kk, Japanese Unexamined Patent Publication H5-25162, Inke, S.A., ES 206819
M. Kawatsura et al., Tetrahedron, 2007, 63, 4172,
T. Kaiho et al., J. Med. Chem., 1989, 32, 351,
Y. Kobayashi et al., Org. Lett., 2005, 7, 183.
Y. Kobayashi et al., Org. Lett., 2005, 7, 1319.

The compounds represented by the general formula (1) according to the present invention can be produced by Synthesis 1 or a combination of conventional methods.

### Synthesis 1

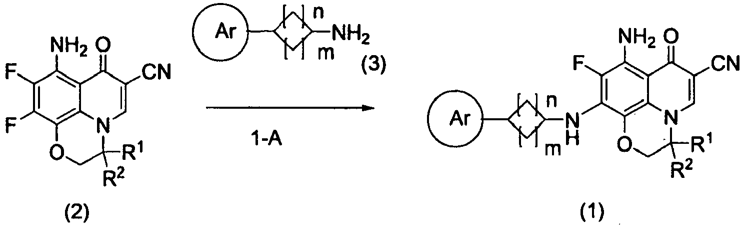

wherein Ar, R¹ R², m and n are as described above.

The conversion from the general formula (2) and the general formula (3) to the general formula (1) (i.e., Process 1-A) is carried out at room temperature to 180°C for 1-48 hours by using a base (such as triethylamine, pyridine, isopropylethylamine, and 1,8-diazabicycloundecyne) in a suitable solvent (such as N,N-dimethylformamide, dimethylsulfoxide, N-methylpyrrolidone, toluene, acetonitrile, tetrahydrofran, methanol, ethanol or a mixture thereof) or solvent-free.

The compounds represented by the general formula (1) according to the present invention can be produced by Synthesis 2 as well.

### Synthesis 2

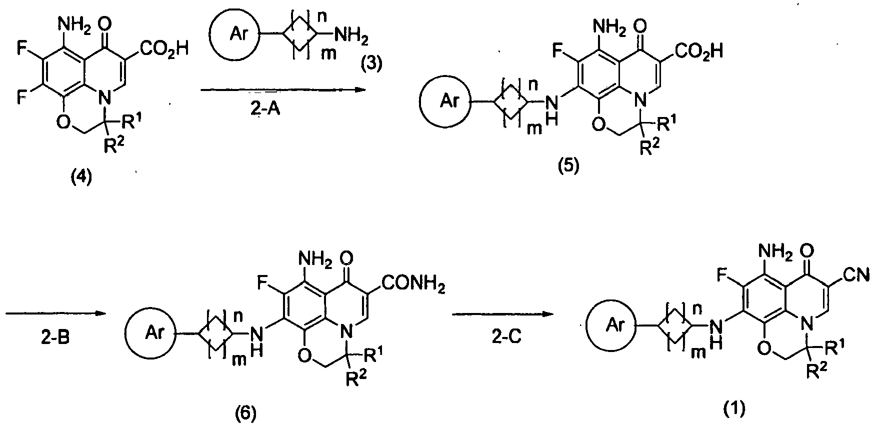

wherein Ar, R¹ R², m and n are as described above.

The conversion from the general formula (4) to the general formula (5) (i.e., Process 2-A) can be performed by a method similar to Process 1-A.

The conversion from the general formula (5) to the general formula (6) (i.e., Process 2-B) can be carried out as follows: the general formula (5) is first reacted with thionyl chloride, thionyl bromide, acetic anhydride, ethyl chlorocarbonate, methyl chlorocarbonate, or the like in a suitable solvent (such as N,N-dimethylformamide, dichloromethane, chloroform, tetrahydrofuran or a mixture thereof) at -15°C to room temperature for 5 min to 3 hours. This converts the carboxyl group to a reactive derivatizing group. The reaction can be carried out in the absence or in the presence of base (such as pyridine and triethylamine). Subsequently, the reaction product is reacted with ammonia aqueous solution in a suitable solvent (such as N,N-dimethylformamide, dichloromethane, chloroform, tetrahydrofuran or a mixture thereof) at a temperature from 0°C to 100°C for 30 min to 24 hours.

The conversion from the general formula (6) to the general formula (1) (i.e., Process 2-C) can be carried out as follows: the general formula (6) is reacted with a dehydroxylation reagent (such as trifluoroacetic anhydride and phosphorus oxychloride) in the presence of base (such as pyridine and triethylamine) in a suitable solvent (such as dichloromethane, chloroform, tetrahydrofuran or a mixture thereof) at -78 °C to 50 °C for 1-24 hours.

The compounds represented by the general formula (3) according to the present invention can be produced by Synthesis 3 as well.

### Synthesis 3

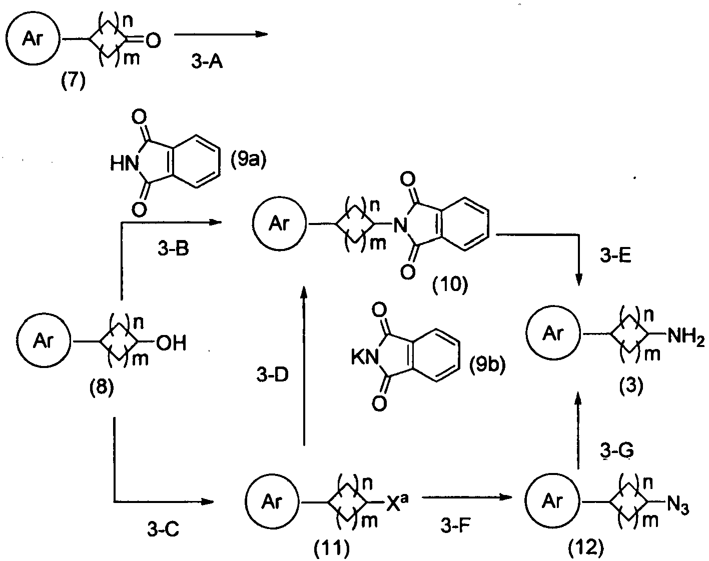

wherein Ar, m and n are as described above, and X^{a} is leaving group (such as halogen atom, p-toluenesulfonyloxy group, methanesulfonyloxy group, trifluomethanesulfonyloxy group).

The conversion from the general formula (7) to the general formula (8) (i.e., Process 3-A) can be carried out at 0°C to 50°C for 30 min to 24 hours by using a reducing reagent (such as sodium borohydride, lithium borohydride, diisobutylaluminum hydride, lithium aluminum hydride) in a suitable solvent (such as tetrahydrofran, methanol, ethanol or a mixture thereof).

The conversion from the general formula (8) and the compound (9a) to the general formula (10) (i.e., Process 3-B) can be carried out at 0°C to 50°C for 1-24 hours by using a Mitsunobu reagent (such as diethyl azodicarboxylate, diisopropyl azodicarboxylate, cyanomethylenetributylphosphorane, N, N, N', N'-tetramethylazodicarboxamide) with phosphine reagent (such as triphenylphosphine, tributylphosphine) if need, in a suitable solvent (such as tetrahydrofran, 1,4-dioxane, tolunene, benzene, or a mixture thereof).

Alternatively, the conversion can be performed as follows: the conversion from the general formula (8) to the general formula (11) (i.e., Process 3-C) can be carried out at 0 °C to room temperature for 1-24 hours by using halogenating agent (such as thionyl chloride, phosphorous oxychloride or thionyl bromide) or sulfonating agent (such as methanesulfonyl chloride, p-toluenesulfonyl chloride or trifluoromethanesulfonic acid anhydride)in the presence of a base (such as triethylamine, pyridine, isopropylethylamine, and 1,8-diazabicycloundecyne) in a suitable solvent (such as N,N-dimethylformamide, dimethylsulfoxide, toluene, acetonitrile, tetrahydrofran, dichloromethene or a mixture thereof).

The conversion from the general formula (11) and the compound (9b) to the general formula (10) (i.e., Process 3-D) can be carried out at 0°C to 100°C for 1-12 hours in a suitable solvent (such as N,N-dimethylformamide, dimethylsulfoxide, N-methylpyrrolidone, toluene, acetonitrile, tetrahydrofran, methanol, ethanol or a mixture thereof).

The conversion from the general formula (10) to the general formula (3) (i.e., Process 3-E) can be carried out at 0°C to 100°C for 30 min-12 hours by using a hydrazine reagent (such as NH₂NH₂-H₂O, MeNHNH₂, PhNHNH₂), acid (such as hydrochloric acid, formic acid) or base (such as KOH, NaOH, K₂CO₃) in a suitable solvent (such as, methanol, ethanol, toluene, acetonitrile, tetrahydrofran, water or a mixture thereof).

Alternatively, the conversion can be performed as follows: the conversion from the general formula (11) to the general formula (12) (i.e., Process 3-F) can be carried out with azidation reagent (such as sodium azide, trimethylsilylazide, diphenylphosphinyl azide) at room temperature to 80°C for 1-24 hours in a suitable solvent (such as dimethylsulfoxide, toluenene, tetrahydrofuran, N,N-dimethylformamide, acetonitrile, dichloromethane or a mixture thereof). The conversion from the general formula (12) to the general formula (3) (i.e., Process 3-G) can performed by hydrogenation using metal catalyst (such as palladium on activated carbon, platinum on activated carbon, oxidized platinum and Raney nickel) in a suitable solvent (such as methanol, ethanol, ethyl acetate, tetrahydrofuran, N,N-dimethylformamide, acetic acid or a mixture thereof) at room temperature to 80°C for 1-24 hours. The reaction can be carried out at a pressure from normal pressure to 0.5 MPa in hydrogen atmosphere.

In Synthesis 3, among the compounds represented by the general formula (7), a compounds represented by the general formula (7a) can be produced by Synthesis 4

### Synthesis 4

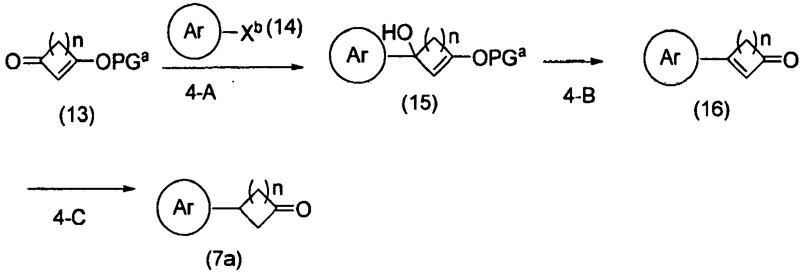

wherein Ar and n are as described above, and PG^{a} is protecting group (such as Methyl, ethyl, benzyl, trimethylsilyl, acetyl) and X^{b} is halogen atom.

The conversion from the general formula (13) and the general formula (14) to the general formula (15) (i.e., Process 4-A) can be performed as follows: the general formula (14) is reacted with alkyl lithium (such as *n*-Butyl lithium, *i*-Butyl lithium, *t*-Butyl lithium, Lithium diisopropylamine) at -78°C to -40°C for 10 min -1 hours in a suitable solvent (such as diethylether, tetrahydrofuran or a mixture thereof), and then the reaction product is reacted with the general formula (13) at -78°C to room temperature for 1-12 hours in a suitable solvent (such as diethylether, tetrahydrofuran or a mixture thereof).

The conversion from the general formula (15) to the general formula (16) (i.e., Process 4-B) can be carried out at -10 to 50°C for 30 min - 12 hours in a suitable solvent (such as water, acetonitrile, tetrahydrofran, dichloromethane, water or a mixture thereof) in the presence of acid (such as hydrochloric acid, trifluoromethneacetic acid, formic acid).

The conversion from the general formula (16) to the general formula (7a) (i.e., Process 4-C) can performed by hydrogenation using metal catalyst (such as palladium on activated carbon, platinum on activated carbon, oxidized platinum and Raney nickel) in a suitable solvent (such as methanol, ethanol, ethyl acetate, tetrahydrofuran, N,N-dimethylformamide, acetic acid or a mixture thereof) at room temperature to 80°C for 1-24 hours. The reaction can be carried out at a pressure from normal pressure to 0.5 MPa in hydrogen atmosphere.

In Synthesis 3, among the compounds represented by the general formula (7), a compounds represented by the general formula (7b) can be produced by Synthesis 5.

### Synthesis 5

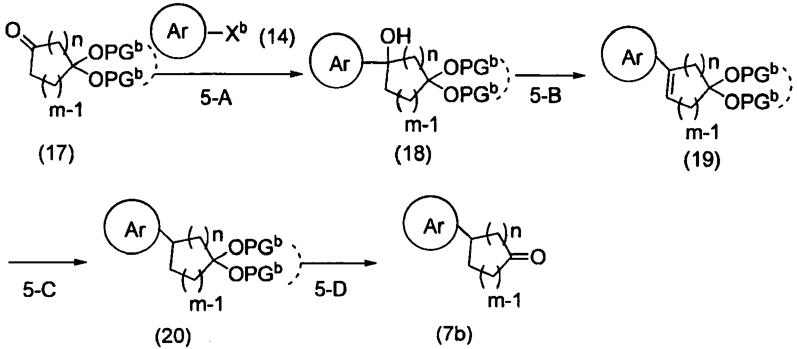

wherein Ar, X^{b} , m and n are as described above, and PG^{b} represents alkyl or aralkyl, or forms a cyclic ketal (such as 1,3-dioxane or 1,3-dioxolane).

The conversion from the general formula (17) and the general formula (14) to the general formula (18) (i.e., Process 5-A) can be performed by a method similar to Process 4-A.

The conversion from the general formula (18) to the general formula (19) (i.e., Process 5-B) can be performed as follows: the general formula (18) is first reacted with halogenating reagent (thionyl chloride, phosphory chloride) or sulfonating reagent (methanesulfonyl chloride, p-toluenesulfonyl chloride) can be carried out at -10°C to room temperature for 30min to 5 hours in the presence of a base (such as triethylamine, pyridine, isopropylethylamine, and 1,8-diazabicycloundecyne) in a suitable solvent (such as toluene, acetonitrile, tetrahydrofran, dichloromethene or a mixture thereof).

Alternatively, the conversion can be carried out at room temperature to 100°C for 30 min to 12 hours in a suitable solvent (such as water, acetonitrile, tetrahydrofran, dichloromethene or a mixture thereof) in the presence of a acid (such as hydrochloric acid, trifluoromethneacetic acid, formic acid).

The conversion from the general formula (19) to the general formula (20) (i.e., Process 5-C) can be performed by a method similar to Process 4-C.

The conversion from the general formula (20) to the general formula (7b) (i.e., Process 5-D) can be carried out at -10 to 50°C for 30 min to 12 hours in a suitable solvent (such as water, acetonitrile, tetrahydrofran, dichloromethane, water or a mixture thereof) in the presence of acid (such as hydrochloric acid, trifluoromethneacetic acid, formic acid).

In Synthesis 3, among the compounds represented by the general formula (8), a compounds represented by the general formula (8a) can be produced by Synthesis 6.

### Synthesis 6

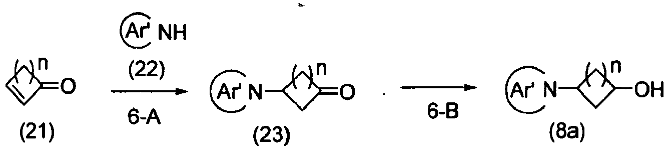

wherein n is as described above, and 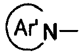 represents pyrroyl, imidazoyl, triazoyl or tetrazoyl which is optionally substituted with one to three substituents.
The conversion from the general formula (21) and the general formula (22) to the general formula (23) (i.e., Process 6-A) can be carried out at 0°C to 50°C for 30 min-24 hours by using a catalyst (such as NaOEt, AcOH, Copper reagent, Lewis acid) in a suitable solvent (such as tetrahydrofran, dichloromethane, toluene, diethylether, ethanol, acetnitrile).
The conversion from the general formula (23) to the general formula (8a) (i.e., Process 6-B) can be performed by a method similar to Process 3-A.
Among the compounds represented by the general formula (3) according to the present invention, the compounds represented by the general formula (3b) and (3c) can be produced by Synthesis 7 as well.

### Synthesis 7

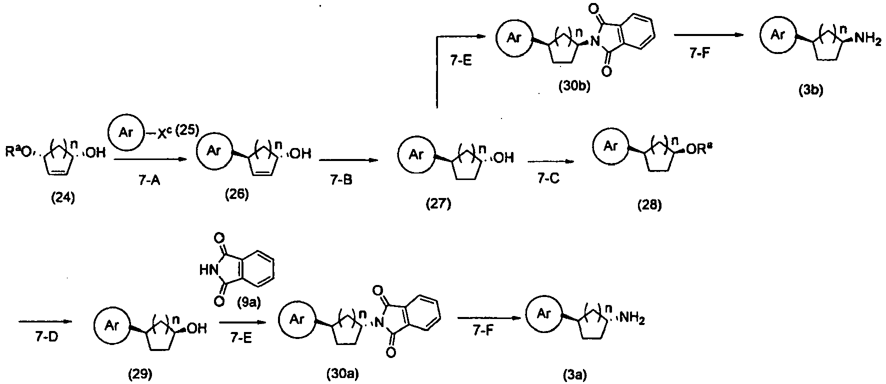

wherein Ar and n are as described above, and X^{c} is halogen atom or halo magnesium (MgCl, MgBr, MgI), and R^{a} is acyl group (such as formyl, acetyl or benzoyl)

The conversion from the general formula (24) and the general formula (25) to the general formula (26) (i.e., Process 7-A) can be performed as follows: when X^{c} represents halogen atom, the general formula (25) is reacted with alkyl lithium (such as n-Butyl lithium, *i*-Butyl lithium, t-Butyl lithium) and Magnesium salt (such as MgCl₂) or Grignard reagent (such as *i*-PrMgCl, *i*-PrMgBr) at -78 °C to room temperature for 1-12 hours in the presence of Copper reagent (such as CuCN, CuI) in the in a suitable solvent (such as diethylether, tetrahydrofuran or a mixture thereof), and then the reaction product is reacted with the general formula (24) at -78°C to room temperature for 1-12 hours in a suitable solvent (such as diethylether, tetrahydrofuran or a mixture thereof).

Alternatively, when X^{c} represents or halo magnesium (MgCl, MgBr, MgI), the general formula (25) is reacted with Copper reagent (such as CuCN, CuI) at -78°C to room temperature for 1-12 hours in a suitable solvent (such as diethylether, tetrahydrofuran or a mixture thereof), and then the reaction product is reacted with the general formula (24) at -78°C to room temperature for 1-12 hours in a suitable solvent (such as diethylether, tetrahydrofuran or a mixture thereof).

The conversion from the general formula (26) to the general formula (27) (i.e., Process 7-B) can be performed by a method similar to Process 4-C.

The conversion from the general formula (27) to the general formula (28) (i.e., Process 7-C) can be carried out by a method similar to Process 3-B with acid reagent (such as acetic acid, trifluoroacetic acid, benzoic acid, p-nitrobenzoic acid).

The conversion from the general formula (28) to the general formula (29) (i.e., Process 7-D) can be carried at 0°C to 50°C for 1-24 hours in the presence of a base (such as K₂CO₃, NaOH, KOH, pyridine) in a suitable solvent (such as methanol, ethanol, tetrahydrofuran, water or a mixture thereof).

The conversion from the general formula (29) to the general formula (30a) or the general formula (27) to the general formula (30b) (i.e., Process 7-E) can be performed by a method similar to Process 3-B.

The conversion from the general formula (30a) to the general formula (3a) or the general formula (30b) to the general formula (3b) (i.e., Process 7-F) can be performed by a method similar to Process 3-E.

In Synthesis 1 and Synthesis 2, the compounds represented by the general formula (2) and (4) can be produced by Synthesis 8.

### Synthesis 8

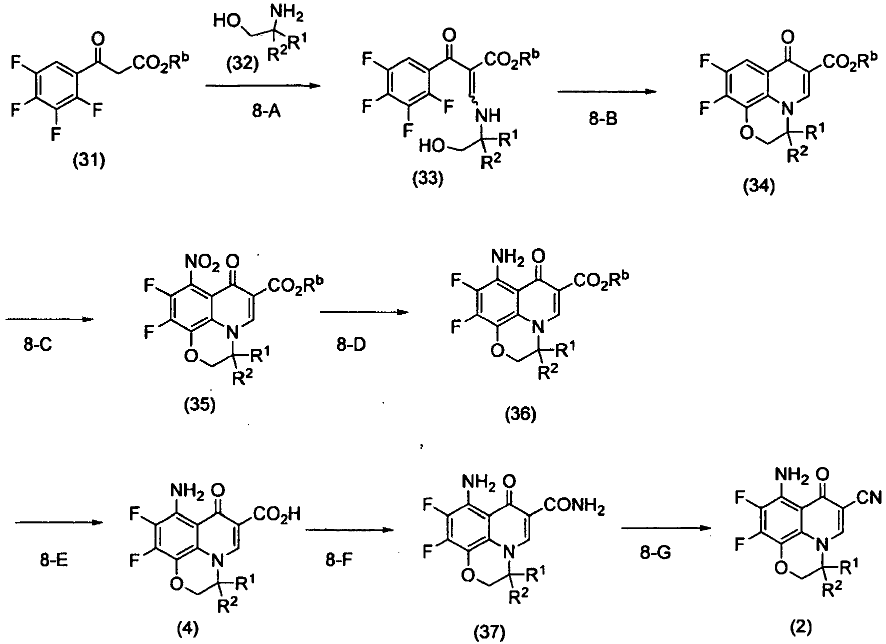

wherein R¹ and R² are as described above, and R^{b} is alkyl (such as methyl, ethyl) .

The conversions from the general formula (31) and the general formula (32) to the general formula (33) (i.e., Process 8-A) can be performed as follows: the general formula (31) is reacted with a mixture of acetic anhydride and ortho-acid ester (such as ortho-formic acid ethyl ester and ortho-acetic ethyl ester) at 100°C to 150°C for 1-8 hours, and then the reaction product is reacted with the general formula (32) in a suitable solvent (such as toluene, tetrahydrofuran, methanol, ethanol, *t*-butanol, or a mixture thereof) at room temperature for 1-24 hours. The reaction can be carried out in the absence or in the presence of a base (potassium carbonate, sodium carbonate, *t*-BuOK, *t*-BuONa, triethylamine and pyridine).

The conversion from the general formula (33) to the general formula (34) (i.e., Process 8-B) can be carried out at 0°C to 150°C for 1-24 hours by using a base (such as potassium hydride, sodium hydride, potassium carbonate, sodium carbonate, *t*-BuOK and *t*-BuONa) or fluoride salt (such as potassium fluoride and sodium fluoride) in a suitable solvent (such as N,N-dimethylformamide, dimethylsulfoxide, tetrahydrofuran, or a mixture thereof).

The conversion from the general formula (34) to the general formula (35) (i.e., Process 8-C) can be conducted by general nitration, for instance, a reaction in concentrated sulfuric acid using a nitrosating reagent (such as potassium nitrate, sodium nitrate and nitric acid). The reaction can be carried out at 0°C to 50°C for 1-8 hours.

The conversion from the general formula (35) to the general formula (36) (i.e., Process 8-D) can be carried out at room temperature to 120° C for 1-48 hours by using a metal (such as reduced iron, tin and zinc) in the presence of acid (such as hydrochloric acid and acetic acid) in a suitable solvent (such as tetrahydrofuran, methanol, ethanol, water or a mixture thereof) or solvent-free.

Alternatively, the conversion can be performed by hydrogenation using metal catalyst (such as palladium on activated carbon, platinum on activated carbon, oxidized platinum and Raney nickel) in a suitable solvent (such as methanol, ethanol, ethyl acetate, tetrahydrofuran, N,N-dimethylformamide, acetic acid or a mixture thereof) at room temperature to 80°C for 1-24 hours. The reaction can be carried out at a pressure from normal pressure to 0.5 MPa in hydrogen atmosphere.

Furthermore, the conversion can be performed by reduction using sodium hydrosulfite in a suitable solvent (such as water, methanol, ethanol, tetrahydrofuran or a mixture thereof) at room temperature to 100°C for 1-24 hours.

The conversion from the general formula (36) to the general formula (4) (i.e., Process 8-E) can be conducted by general hydrolysis, for instance, a reaction with alkali (such as KOH, NaOH, LiOH) or acid (such as hydrochloric acid, sulfuric acid, trifluoromethaneacetic acid) in a suitable solvent (such as water, methanol, ethanol, tetrahydrofuran or a mixture thereof). The reaction can be carried out at 0°C to 50°C for 1-8 hours.

The conversion from the general formula (4) to the general formula (37) (i.e., Process 8-F) can be performed by a method similar to Process 2-B.

The conversion from the general formula (37) to the general formula (2) (i.e., Process 8-G) can be performed by a method similar to Process 2-C.

Optical isomers of compounds represented by the general formula (1) may be synthesized using optically active material compounds according to the aforementioned Synthesis 1 to 3 and 7.

Racemic compounds represented by the general formula (1) may be synthesized by separation and recrystallization using optically active acid or base.

It may be produced by a chromatographic technique using a choral support.

### D. Pharmaceutical Compositions

The pharmaceutical compositions provided herein contain therapeutically effective amounts of one or more of compounds provided herein that are useful in the prevention, treatment, or amelioration of one or more of the symptoms of GSK-3 mediated diseases.

Pharmaceutical compositions of the compounds provided herein may be administered systemically or locally, or orally or parentelly such as rectally, subcutaneously, intramuscularly, intravenously or percutaneusly.

The compositions contain one or more compounds provided herein. The compounds can be formulated into suitable pharmaceutical preparations such as solutions, suspensions, tablets, dispersible tablets, pills, capsules, granules, powders, fine powders, injections, sustained release formulations or elixirs, for oral administration or in sterile solutions or suspensions for parenteral administration, as well as transdermal patch preparation and dry powder inhalers. In certain embodiments, the compositions provided herein can be produced by adding convenient excipients, fillers, binders, disintegrators, coating agents, sugar coating agents, pH modulators, solublizing agents, or aqueous or non-aqueous solvents according to the conventional pharmaceutical preparation techniques. Typically, the compounds described above are formulated into pharmaceutical compositions using techniques and procedures well known in the art (see, e.g., Ansel Introduction to Pharmaceutical Dosage Forms, Seventh Edition 1999).

In the compositions, effective concentrations of one or more compounds or pharmaceutically acceptable salts is (are) mixed with a suitable pharmaceutical carrier or vehicle. The compounds may be derivatized as the corresponding salts, esters, enol ethers or esters, acids, bases, solvates, hydrates or prodrugs prior to formulation, as described above. The concentrations of the compounds in the compositions are effective for delivery of an amount, upon administration, that treats, prevents, or ameliorates one or more of the symptoms of GSK-3 mediated diseases.

Typically, the compositions are formulated for single dosage administration. To formulate a composition, the weight fraction of compound is dissolved, suspended, dispersed or otherwise mixed in a selected vehicle at an effective concentration such that the treated condition is relieved or ameliorated. Pharmaceutical carriers or vehicles suitable for administration of the compounds provided herein include any such carriers known to those skilled in the art to be suitable for the particular mode of administration.

In addition, the compounds may be formulated as the sole pharmaceutically active ingredient in the composition or may be combined with other active ingredients. Liposomal suspensions, including tissue-targeted liposomes, such as tumor-targeted liposomes, may also be suitable as pharmaceutically acceptable carriers. These may be prepared according to methods known to those skilled in the art. For example, liposome formulations may be prepared as known in the art. Briefly, liposomes such as multilamellar vesicles (MLV's) may be formed by drying down egg phosphatidyl choline and brain phosphatidyl serine (7:3 molar ratio) on the inside of a flask. A solution of a compound provided herein in phosphate buffered saline lacking divalent cations (PBS) is added and the flask shaken until the lipid film is dispersed. The resulting vesicles are washed to remove unencapsulated compound, pelleted by centrifugation, and then resuspended in PBS.

The active compound is included in the pharmaceutically acceptable carrier in an amount sufficient to exert a therapeutically useful effect in the absence of undesirable side effects on the patient treated. The therapeutically effective concentration may be determined empirically by testing the compounds in *in vitro* and *in vivo* systems described herein and then extrapolated therefrom for dosages for humans.

The concentration of active compound in the pharmaceutical composition will depend on absorption, inactivation and excretion rates of the active compound, the physicochemical characteristics of the compound, the dosage schedule, and amount administered as well as other factors known to those of skill in the art. For example, the amount that is delivered is sufficient to ameliorate one or more of the symptoms of GSK-3 mediated diseases.

In certain embodiments, a therapeutically effective dosage should produce a serum concentration of active ingredient of from about 0.1 ng/ml to about 50-100 µg/ml. In one embodiment, the pharmaceutical compositions provide a dosage of from about 0.001 mg to about 2000 mg of compound per kilogram of body weight per day. Pharmaceutical dosage unit forms are prepared to provide from about 1 mg to about 1000 mg and in certain embodiments, from about 10 to about 500 mg of the essential active ingredient or a combination of essential ingredients per dosage unit form.

The active ingredient may be administered at once, or may be divided into a number of smaller doses to be administered at intervals of time. It is understood that the precise dosage and duration of treatment is a function of the disease being treated and may be determined empirically using known testing protocols or by extrapolation from *in vivo* or *in vitro* test data. It is to be noted that concentrations and dosage values may also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed compositions.

Thus, effective concentrations or amounts of one or more of the compounds described herein or pharmaceutically acceptable salts thereof are mixed with a suitable pharmaceutical carrier or vehicle for systemic, topical or local administration to form pharmaceutical compositions. Compounds are included in an amount effective for ameliorating one or more symptoms of, or for treating or preventing kinase-mediated, including, but not limited to, GSK-3-mediated diseases. The concentration of active compound in the composition will depend on absorption, inactivation, excretion rates of the active compound, the dosage schedule, amount administered, particular formulation as well as other factors known to those of skill in the art.

The compositions are intended to be administered by a suitable route, including orally, parenterally, rectally, topically and locally. For oral administration, capsules and tablets can be formulated. The compositions are in liquid, semi-liquid or solid form and are formulated in a manner suitable for each route of administration.

Solutions or suspensions used for parenteral, intradermal, subcutaneous, or topical application can include any of the following components: a sterile diluent, such as water for injection, saline solution, fixed oil, polyethylene glycol, glycerine, propylene glycol, dimethyl acetamide or other synthetic solvent; antimicrobial agents, such as benzyl alcohol and methyl parabens; antioxidants, such as ascorbic acid and sodium bisulfite; chelating agents, such as ethylenediaminetetraacetic acid (EDTA); buffers, such as acetates, citrates and phosphates; and agents for the adjustment of tonicity such as sodium chloride or dextrose. Parenteral preparations can be enclosed in ampules, disposable syringes or single or multiple dose vials made of glass, plastic or other suitable material.

In instances in which the compounds exhibit insufficient solubility, methods for solubilizing compounds may be used. Such methods are known to those of skill in this art, and include, but are not limited to, using cosolvents, such as dimethylsulfoxide (DMSO), using surfactants, such as TWEEN®, or dissolution in aqueous sodium bicarbonate.

Upon mixing or addition of the compound(s), the resulting mixture may be a solution, suspension, emulsion or the like. The form of the resulting mixture depends upon a number of factors, including the intended mode of administration and the solubility of the compound in the selected carrier or vehicle. The effective concentration is sufficient for ameliorating the symptoms of the disease, disorder or condition treated and may be empirically determined.

The pharmaceutical compositions are provided for administration to humans and animals in unit dosage forms, such as tablets, capsules, pills, powders, granules, sterile parenteral solutions or suspensions, and oral solutions or suspensions, and oil water emulsions containing suitable quantities of the compounds or pharmaceutically acceptable salts thereof. The pharmaceutically therapeutically active compounds and salts thereof are formulated and administered in unit dosage forms or multiple dosage forms. Unit dose forms as used herein refer to physically discrete units suitable for human and animal subjects and packaged individually as is known in the art. Each unit dose contains a predetermined quantity of the therapeutically active compound sufficient to produce the desired therapeutic effect, in association with the required pharmaceutical carrier, vehicle or diluent. Examples of unit dose forms include ampules and syringes and individually packaged tablets or capsules. Unit dose forms may be administered in fractions or multiples thereof. A multiple dose form is a plurality of identical unit dosage forms packaged in a single container to be administered in segregated unit dose form. Examples of multiple dose forms include vials, bottles of tablets or capsules or bottles of pints or gallons. Hence, multiple dose form is a multiple of unit doses which are not segregated in packaging.

Sustained-release preparations can also be prepared. Suitable examples of sustained-release preparations include semipermeable matrices of solid hydrophobic polymers containing the compound provided herein, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides, copolymers of L-glutamic acid and ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated compound remain in the body for a long time, they may denature or aggregate as a result of exposure to moisture at 37 °C, resulting in a loss of biological activity and possible changes in their structure. Rational strategies can be devised for stabilization depending on the mechanism of action involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

Dosage forms or compositions containing active ingredient in the range of 0.005% to 100% with the balance made up from non toxic carrier may be prepared. For oral administration, a pharmaceutically acceptable non toxic composition is formed by the incorporation of any of the normally employed excipients, such as, for example pharmaceutical grades of mannitol, lactose, starch, magnesium stearate, talcum or talc, cellulose derivatives, gelatin, agar, pectin, arabic gum, olive oil, sesame oil, cacao butter, ethylene glycol, sodium crosscarmellose, glucose, sucrose, magnesium carbonate or sodium saccharin. Such compositions include solutions, suspensions, tablets, capsules, powders and sustained release formulations, such as, but not limited to, implants and microencapsulated delivery systems, and biodegradable, biocompatible polymers, such as collagen, ethylene vinyl acetate, polyanhydrides, polyglycolic acid, polyorthoesters, polylactic acid and others. Methods for preparation of these compositions are known to those skilled in the art. The contemplated compositions may contain about 0.001-100% active ingredient, in certain embodiments, about 0.1-85% or about 75-95%.

The active compounds or pharmaceutically acceptable salts may be prepared with carriers that protect the compound against rapid elimination from the body, such as time release formulations or coatings.

The compositions may include other active compounds to obtain desired combinations of properties. The compounds provided herein, or pharmaceutically acceptable salts thereof as described herein, may also be advantageously administered for therapeutic or prophylactic purposes together with another pharmacological agent known in the general art to be of value in treating one or more of the diseases or medical conditions referred to hereinabove, such as GSK-3 mediated diseases. It is to be understood that such combination therapy constitutes a further aspect of the compositions and methods of treatment provided herein.

Lactose-free compositions provided herein can contain excipients that are well known in the art and are listed, for example, in the U.S. Pharmocopia (USP) SP (XXI)/NF (XVI). In general, lactose-free compositions contain an active ingredient, a binder/filler, and a lubricant in pharmaceutically compatible and pharmaceutically acceptable amounts. Exemplary lactose-free dosage forms contain an active ingredient, microcrystalline cellulose, pre-gelatinized starch and magnesium stearate.

Further encompassed are anhydrous pharmaceutical compositions and dosage forms containing a compound provided herein. For example, the addition of water (e.g., 5%) is widely accepted in the pharmaceutical arts as a means of simulating long-term storage in order to determine characteristics such as shelf-life or the stability of formulations over time. *See*, *e.g*., Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment and use of formulations.

Anhydrous pharmaceutical compositions and dosage forms as described herein can be prepared using anhydrous or low moisture containing ingredients and low moisture or low humidity conditions. Pharmaceutical compositions and dosage forms that comprise lactose and at least one active ingredient that comprises a primary or secondary amine may be, in certain embodiments, anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are, in certain embodiments, packaged using materials known to prevent exposure to water such that they can be included in suitable formulary kits. Examples of suitable packaging include, but are not limited to, hermetically sealed foils, plastics, unit dose containers (*e.g*., vials), blister packs and strip packs.

### Oral Dosage Forms

Oral pharmaceutical dosage forms are either solid, gel or liquid. The solid dosage forms are tablets, capsules, granules, and bulk powders. Types of oral tablets include compressed, chewable lozenges and tablets which may be enteric coated, sugar coated or film coated. Capsules may be hard or soft gelatin capsules, while granules and powders may be provided in non effervescent or effervescent form with the combination of other ingredients known to those skilled in the art.

In certain embodiments, the formulations are solid dosage forms, such as capsules or tablets. The tablets, pills, capsules, troches and the like can contain any of the following ingredients, or compounds of a similar nature: a binder; a diluent; a disintegrating agent; a lubricant; a glidant; a sweetening agent; and a flavoring agent.

Examples of binders include microcrystalline cellulose, gum tragacanth, glucose solution, acacia mucilage, gelatin solution, sucrose and starch paste. Lubricants include talc, starch, magnesium or calcium stearate, lycopodium and stearic acid. Diluents include, for example, lactose, sucrose, starch, kaolin, salt, mannitol and dicalcium phosphate. Glidants include, but are not limited to, colloidal silicon dioxide. Disintegrating agents include crosscarmellose sodium, sodium starch glycolate, alginic acid, corn starch, potato starch, bentonite, methylcellulose, agar and carboxymethylcellulose. Coloring agents include, for example, any of the approved certified water soluble FD and C dyes, mixtures thereof; and water insoluble FD and C dyes suspended on alumina hydrate. Sweetening agents include sucrose, lactose, mannitol and artificial sweetening agents such as saccharin, and any number of spray dried flavors. Flavoring agents include natural flavors extracted from plants such as fruits and synthetic blends of compounds which produce a pleasant sensation, such as, but not limited to peppermint and methyl salicylate. Wetting agents include propylene glycol monostearate, sorbitan monooleate, diethylene glycol monolaurate and polyoxyethylene laural ether. Emetic coatings include fatty acids, fats, waxes, shellac, ammoniated shellac and cellulose acetate phthalates. Film coatings include hydroxyethylcellulose, sodium carboxymethylcellulose, polyethylene glycol 4000 and cellulose acetate phthalate.

If oral administration is desired, the compound could be provided in a composition that protects it from the acidic environment of the stomach. For example, the composition can be formulated in an enteric coating that maintains its integrity in the stomach and releases the active compound in the intestine. The composition may also be formulated in combination with an antacid or other such ingredient.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar and other enteric agents. The compounds can also be administered as a component of an elixir, suspension, syrup, wafer, sprinkle, chewing gum or the like. Syrup may contain, in addition to the active compounds, sucrose as a sweetening agent and certain preservatives, dyes and colorings and flavors.

The active materials can also be mixed with other active materials which do not impair the desired action, or with materials that supplement the desired action, such as antacids, H2 blockers, and diuretics. The active ingredient is a compound or pharmaceutically acceptable salt thereof as described herein. Higher concentrations, up to about 98% by weight of the active ingredient may be included.

Pharmaceutically acceptable carriers included in tablets are binders, lubricants, diluents, disintegrating agents, coloring agents, flavoring agents, and wetting agents. Enteric coated tablets, because of the enteric coating, resist the action of stomach acid and dissolve or disintegrate in the neutral or alkaline intestines. Sugar coated tablets are compressed tablets to which different layers of pharmaceutically acceptable substances are applied. Film coated tablets are compressed tablets which have been coated with a polymer or other suitable coating. Multiple compressed tablets are compressed tablets made by more than one compression cycle utilizing the pharmaceutically acceptable substances previously mentioned. Coloring agents may also be used in the above dosage forms. Flavoring and sweetening agents are used in compressed tablets, sugar coated, multiple compressed and chewable tablets. Flavoring and sweetening agents are especially useful in the formation of chewable tablets and lozenges.

Liquid oral dosage forms include aqueous solutions, emulsions, suspensions, solutions and/or suspensions reconstituted from non effervescent granules and effervescent preparations reconstituted from effervescent granules. Aqueous solutions include, for example, elixirs and syrups. Emulsions are either oil in-water or water in oil.

Elixirs are clear, sweetened, hydroalcoholic preparations. Pharmaceutically acceptable carriers used in elixirs include solvents. Syrups are concentrated aqueous solutions of a sugar, for example, sucrose, and may contain a preservative. An emulsion is a two phase system in which one liquid is dispersed in the form of small globules throughout another liquid. Pharmaceutically acceptable carriers used in emulsions are non aqueous liquids, emulsifying agents and preservatives. Suspensions use pharmaceutically acceptable suspending agents and preservatives. Pharmaceutically acceptable substances used in non effervescent granules, to be reconstituted into a liquid oral dosage form, include diluents, sweeteners and wetting agents. Pharmaceutically acceptable substances used in effervescent granules, to be reconstituted into a liquid oral dosage form, include organic acids and a source of carbon dioxide. Coloring and flavoring agents are used in all of the above dosage forms.

Solvents include glycerin, sorbitol, ethyl alcohol and syrup. Examples of preservatives include glycerin, methyl and propylparaben, benzoic add, sodium benzoate and alcohol. Examples of non aqueous liquids utilized in emulsions include mineral oil and cottonseed oil. Examples of emulsifying agents include gelatin, acacia, tragacanth, bentonite, and surfactants such as polyoxyethylene sorbitan monooleate. Suspending agents include sodium carboxymethylcellulose, pectin, tragacanth, Veegum and acacia. Diluents include lactose and sucrose. Sweetening agents include sucrose, syrups, glycerin and artificial sweetening agents such as saccharin. Wetting agents include propylene glycol monostearate, sorbitan monooleate, diethylene glycol monolaurate and polyoxyethylene lauryl ether. Organic adds include citric and tartaric acid. Sources of carbon dioxide include sodium bicarbonate and sodium carbonate. Coloring agents include any of the approved certified water soluble FD and C dyes, and mixtures thereof. Flavoring agents include natural flavors extracted from plants such as fruits, and synthetic blends of compounds which produce a pleasant taste sensation.

For a solid dosage form, the solution or suspension, in for example propylene carbonate, vegetable oils or triglycerides, is encapsulated in a gelatin capsule. Such solutions, and the preparation and encapsulation thereof, are disclosed in U.S. Patent Nos 4,328,245; 4,409,239; and 4,410,545. For a liquid dosage form, the solution, e.g., for example, in a polyethylene glycol, may be diluted with a sufficient quantity of a pharmaceutically acceptable liquid carrier, e.g., water, to be easily measured for administration.

Alternatively, liquid or semi solid oral formulations may be prepared by dissolving or dispersing the active compound or salt in vegetable oils, glycols, triglycerides, propylene glycol esters (e.g., propylene carbonate) and other such carriers, and encapsulating these solutions or suspensions in hard or soft gelatin capsule shells. Other useful formulations include, but are not limited to, those containing a compound provided herein, a dialkylated mono- or poly-alkylene glycol, including, but not limited to, 1,2-dimethoxymethane, diglyme, triglyme, tetraglyme, polyethylene glycol-350-dimethyl ether, polyethylene glycol-550-dimethyl ether, polyethylene glycol-750-dimethyl ether wherein 350, 550 and 750 refer to the approximate average molecular weight of the polyethylene glycol, and one or more antioxidants, such as butylated hydroxytoluene (BHT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, thiodipropionic acid and its esters, and dithiocarbamates.

Other formulations include, but are not limited to, aqueous alcoholic solutions including a pharmaceutically acceptable acetal. Alcohols used in these formulations are any pharmaceutically acceptable water-miscible solvents having one or more hydroxyl groups, including, but not limited to, propylene glycol and ethanol. Acetals include, but are not limited to, di(lower alkyl) acetals of lower alkyl aldehydes such as acetaldehyde diethyl acetal.

In all embodiments, tablets and capsules formulations may be coated as known by those of skill in the art in order to modify or sustain dissolution of the active ingredient. Thus, for example, they may be coated with a conventional enterically digestible coating, such as phenylsalicylate, waxes and cellulose acetate phthalate.

### Injectables, solutions and emulsions

Parenteral administration, generally characterized by injection, either subcutaneously, intramuscularly or intravenously is also contemplated herein. Injectables can be prepared in conventional forms, either as liquid solutions or suspensions, solid forms suitable for solution or suspension in liquid prior to injection, or as emulsions. Suitable excipients are, for example, water, saline, dextrose, glycerol or ethanol. In addition, if desired, the pharmaceutical compositions to be administered may also contain minor amounts of non toxic auxiliary substances such as wetting or emulsifying agents, pH buffering agents, stabilizers, solubility enhancers, and other such agents, such as for example, sodium acetate, sorbitan monolaurate, triethanolamine oleate and cyclodextrins. Implantation of a slow release or sustained release system, such that a constant level of dosage is maintained is also contemplated herein. Briefly, a compound provided herein is dispersed in a solid inner matrix, e.g., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, e.g., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The compound diffuses through the outer polymeric membrane in a release rate controlling step. The percentage of active compound contained in such parenteral compositions is highly dependent on the specific nature thereof, as well as the activity of the compound and the needs of the subject.

Parenteral administration of the compositions includes intravenous, subcutaneous and intramuscular administrations. Preparations for parenteral administration include sterile solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use and sterile emulsions. The solutions may be either aqueous or nonaqueous.

If administered intravenously, suitable carriers include physiological saline or phosphate buffered saline (PBS), and solutions containing thickening and solubilizing agents, such as glucose, polyethylene glycol, and polypropylene glycol and mixtures thereof.

Pharmaceutically acceptable carriers used in parenteral preparations include aqueous vehicles, nonaqueous vehicles, antimicrobial agents, isotonic agents, buffers, antioxidants, local anesthetics, suspending and dispersing agents, emulsifying agents, sequestering or chelating agents and other pharmaceutically acceptable substances.

Examples of aqueous vehicles include Sodium Chloride Injection, Ringers Injection, Isotonic Dextrose Injection, Sterile Water Injection, Dextrose and Lactated Ringers Injection. Nonaqueous parenteral vehicles include fixed oils of vegetable origin, cottonseed oil, corn oil, sesame oil and peanut oil. Antimicrobial agents in bacteriostatic or fungistatic concentrations must be added to parenteral preparations packaged in multiple dose containers which include phenols or cresols, mercurials, benzyl alcohol, chlorobutanol, methyl and propyl p hydroxybenzoic acid esters, thimerosal, benzalkonium chloride and benzethonium chloride. Isotonic agents include sodium chloride and dextrose. Buffers include phosphate and citrate. Antioxidants include sodium bisulfate. Local anesthetics include procaine hydrochloride. Suspending and dispersing agents include sodium carboxymethylcelluose, hydroxypropyl methylcellulose and polyvinylpyrrolidone. Emulsifying agents include Polysorbate 80 (TWEEN® 80). A sequestering or chelating agent of metal ions includes EDTA. Pharmaceutical carriers also include ethyl alcohol, polyethylene glycol and propylene glycol for water miscible vehicles and sodium hydroxide, hydrochloric acid, citric acid or lactic acid for pH adjustment.

The concentration of the pharmaceutically active compound is adjusted so that an injection provides an effective amount to produce the desired pharmacological effect. The exact dose depends on the age, weight and condition of the patient or animal as is known in the art.

The unit dose parenteral preparations are packaged in an ampule, a vial or a syringe with a needle. All preparations for parenteral administration must be sterile, as is known and practiced in the art.

Illustratively, intravenous or intraarterial infusion of a sterile aqueous solution containing an active compound is an effective mode of administration. Another embodiment is a sterile aqueous or oily solution or suspension containing an active material injected as necessary to produce the desired pharmacological effect.

Injectables are designed for local and systemic administration. Typically a therapeutically effective dosage is formulated to contain a concentration of at least about 0.1 % w/w up to about 90% w/w or more, such as more than 1% w/w of the active compound to the treated tissue(s). The active ingredient may be administered at once, or may be divided into a number of smaller doses to be administered at intervals of time. It is understood that the precise dosage and duration of treatment is a function of the tissue being treated and may be determined empirically using known testing protocols or by extrapolation from *in vivo* or *in vitro* test data. It is to be noted that concentrations and dosage values may also vary with the age of the individual treated. It is to be further understood that for any particular subject, specific dosage regimens should be adjusted over time according to the individual need and the professional judgment of the person administering or supervising the administration of the formulations, and that the concentration ranges set forth herein are exemplary only and are not intended to limit the scope or practice of the claimed formulations.

The compound may be suspended in micronized or other suitable form or may be derivatized to produce a more soluble active product or to produce a prodrug. The form of the resulting mixture depends upon a number of factors, including the intended mode of administration and the solubility of the compound in the selected carrier or vehicle. The effective concentration is sufficient for ameliorating the symptoms of the condition and may be empirically determined.

### Lyonhilized powders

Of interest herein are also lyophilized powders, which can be reconstituted for administration as solutions, emulsions and other mixtures. They may also be reconstituted and formulated as solids or gels.

The sterile, lyophilized powder is prepared by dissolving a compound provided herein, or a pharmaceutically acceptable salt thereof, in a suitable solvent. The solvent may contain an excipient which improves the stability or other pharmacological component of the powder or reconstituted solution, prepared from the powder. Excipients that may be used include, but are not limited to, dextrose, sorbital, fructose, corn syrup, xylitol, glycerin, glucose, sucrose or other suitable agent. The solvent may also contain a buffer, such as citrate, sodium or potassium phosphate or other such buffer known to those of skill in the art at, in one embodiment, about neutral pH. Subsequent sterile filtration of the solution followed by lyophilization under standard conditions known to those of skill in the art provides the desired formulation. Generally, the resulting solution will be apportioned into vials for lyophilization. Each vial will contain a single dosage (i.e., 10-1000 mg or 100-500 mg) or multiple dosages of the compound. The lyophilized powder can be stored under appropriate conditions, such as at about 4 °C to room temperature.

Reconstitution of this lyophilized powder with water for injection provides a formulation for use in parenteral administration. For reconstitution, about 1-50 mg, about 5-35 mg, or about 9-30 mg of lyophilized powder, is added per mL of sterile water or other suitable carrier. The precise amount depends upon the selected compound. Such amount can be empirically determined.

### Topical administration

Topical mixtures are prepared as described for the local and systemic administration. The resulting mixture may be a solution, suspension, emulsions or the like and are formulated as creams, gels, ointments, emulsions, solutions, elixirs, lotions, suspensions, tinctures, pastes, foams, aerosols, irrigations, sprays, suppositories, bandages, dermal patches or any other formulations suitable for topical administration.

The compounds or pharmaceutically acceptable salts thereof may be formulated as aerosols for topical application, such as by inhalation (see, e.g., U.S. Patent Nos. 4,044,126, 4,414,209, and 4,364,923 which describe aerosols for delivery of a steroid useful for treatment of inflammatory diseases, particularly asthma). These formulations for administration to the respiratory tract can be in the form of an aerosol or solution for a nebulizer, or as a microfine powder for insufflation, alone or in combination with an inert carrier such as lactose. In such a case, the particles of the formulation will have diameters of less than 50 microns or less than 10 microns.

The compounds may be formulated for local or topical application, such as for topical application to the skin and mucous membranes, such as in the eye, in the form of gels, creams, and lotions and for application to the eye or for intracisternal or intraspinal application. Topical administration is contemplated for transdermal delivery and also for administration to the eyes or mucosa, or for inhalation therapies. Nasal solutions of the active compound alone or in combination with other pharmaceutically acceptable excipients can also be administered.

These solutions, particularly those intended for ophthalmic use, may be formulated as 0.01% - 10% isotonic solutions, pH about 5-7, with appropriate salts.

### Compositions for other routes of administration

Other routes of administration, such as topical application, transdermal patches, and rectal administration are also contemplated herein.

For example, pharmaceutical dosage forms for rectal administration are rectal suppositories, capsules and tablets for systemic effect. Rectal suppositories are used herein mean solid bodies for insertion into the rectum which melt or soften at body temperature releasing one or more pharmacologically or therapeutically active ingredients. Pharmaceutically acceptable substances utilized in rectal suppositories are bases or vehicles and agents to raise the melting point. Examples of bases include cocoa butter (theobroma oil), glycerin gelatin, carbowax (polyoxyethylene glycol) and appropriate mixtures of mono , di and triglycerides of fatty acids. Combinations of the various bases may be used. Agents to raise the melting point of suppositories include spermaceti and wax. Rectal suppositories may be prepared either by the compressed method or by molding. An exemplary weight of a rectal suppository is about 2 to 3 gm.

Tablets and capsules for rectal administration are manufactured using the same pharmaceutically acceptable substance and by the same methods as for formulations for oral administration.

### Sustained Release Compositions

Active ingredients provided herein can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, 5,639,480, 5,733,566, 5,739,108, 5,891,474, 5,922,356, 5,972,891, 5,980,945, 5,993,855, 6,045,830, 6,087,324, 6,113,943, 6,197,350, 6,248,363, 6,264,970, 6,267,981, 6,376,461,6,419,961, 6,589,548, 6,613,358, 6,699,500 and 6,740,634. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein can be readily selected for use with the active ingredients provided herein.

All controlled-release pharmaceutical products have a common goal of improving drug therapy over that achieved by their non-controlled counterparts. In one embodiment, the use of an optimally designed controlled-release preparation in medical treatment is characterized by a minimum of drug substance being employed to cure or control the condition in a minimum amount of time. In certain embodiments, advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g*., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

In certain embodiments, the agent may be administered using intravenous infusion, an implantable osmotic pump, a transdermal patch, liposomes, or other modes of administration. In one embodiment, a pump may be used (see, Sefton, CRC Crit. Ref Biomed. Eng. 14:201 (1987); Buchwald et al., Surgery 88:507 (1980); Saudek et al., N. Engl. J. Med. 321:574 (1989). In another embodiment, polymeric materials can be used. In yet another embodiment, a controlled release system can be placed in proximity of the therapeutic target, i.e., thus requiring only a fraction of the systemic dose (see, *e.g*., Goodson, Medical Applications of Controlled Release, vol. 2, pp. 115-138 (1984).

In some embodiments, a controlled release device is introduced into a subject in proximity of the site of inappropriate immune activation or a tumor. Other controlled release systems are discussed in the review by Langer (Science 249:1527-1533 (1990). The active ingredient can be dispersed in a solid inner matrix, *e.g*., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, *e.g*., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The active ingredient then diffuses through the outer polymeric membrane in a release rate controlling step. The percentage of active ingredient contained in such parenteral compositions is highly dependent on the specific nature thereof, as well as the needs of the subject.

### Targeted Formulations

The compounds provided herein, or pharmaceutically acceptable salts thereof, may also be formulated to be targeted to a particular tissue, receptor, or other area of the body of the subject to be treated. Many such targeting methods are well known to those of skill in the art. All such targeting methods are contemplated herein for use in the instant compositions. For non-limiting examples of targeting methods, see, e.g., U.S. Patent Nos. 6,316,652, 6,274,552, 6,271,359, 6,253,872, 6,139,865, 6,131,570, 6,120,751, 6,071,495, 6,060,082, 6,048,736, 6,039,975, 6,004,534, 5,985,307, 5,972,366, 5,900,252, 5,840,674, 5,759,542 and 5,709,874.

In one embodiment, liposomal suspensions, including tissue-targeted liposomes, such as tumor-targeted liposomes, may also be suitable as pharmaceutically acceptable carriers. These may be prepared according to methods known to those skilled in the art. For example, liposome formulations may be prepared as described in U.S. Patent No. 4,522,811. Briefly, liposomes such as multilamellar vesicles (MLV's) may be formed by drying down egg phosphatidyl choline and brain phosphatidyl serine (7:3 molar ratio) on the inside of a flask. A solution of a compound provided herein in phosphate buffered saline lacking divalent cations (PBS) is added and the flask shaken until the lipid film is dispersed. The resulting vesicles are washed to remove unencapsulated compound, pelleted by centrifugation, and then resuspended in PBS.

### Articles of manufacture

The compounds or pharmaceutically acceptable salts can be packaged as articles of manufacture containing packaging material, a compound or pharmaceutically acceptable salt thereof provided herein, which is used for treatment, prevention or amelioration of one or more symptoms associated with kinase activity, including, but not limited to, GSK-3 activity, and a label that indicates that the compound or pharmaceutically acceptable salt thereof is used for treatment, prevention or amelioration of one or more symptoms of kinase-mediated, including, but not limited to, GSK-3-mediated diseases.

The articles of manufacture provided herein contain packaging materials. Packaging materials for use in packaging pharmaceutical products are well known to those of skill in the art. See, e.g., U.S. Patent Nos. 5,323,907, 5,052,558 and 5,033,252. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. A wide array of formulations of the compounds and compositions provided herein are contemplated.

### E. Methods of Treatment

Methods of use of the compounds and compositions are also described. The methods involve both *in vitro* and *in vivo* uses of the compounds and compositions.

In certain embodiments, described herein are methods for inhibiting the action of GSK-3 by administering compounds and compositions provided herein. In one embodiment, the methods involve contacting GSK-3 with a compound provided herein.

### F. Evaluation of Compound Activity

### A) GSK inhibition

GSK3 inhibitory activity of the compounds provided herein can be readily detected using the assays described herein, as well as assays generally known to those of ordinary skill in the art.

Exemplary methods for identifying specific inhibitors of GSK3 include both cell-free and cell-based GSK3 kinase assays. A cell-free GSK3 kinase assay detects inhibitors that act by direct interaction with the polypeptide GSK3, while a cell-based GSK3 kinase assay may identify inhibitors that function either by direct interaction with GSK3 itself, or by interference with GSK3 expression or with post-translational processing required to produce mature active GSK3. U.S. Application No. 20050054663 describes exemplary cell-free and cell-based GSK3 kinase assays. Exemplary assays used herein are discussed briefly below:
10-25 ng of recombinant full-length human GSK3β (Upstate) is incubated in the presence or absence of compound at varying concentrations for 1 hour at 30 degrees Celsius in 20 mM MOPS, pH 7.0, 10 mM magnesium acetate, 0.2 mM EDTA, 2 mM EGTA, 30 mM magnesium chloride, 62.5 µM phospho-glycogen synthase peptide-2, 5µM ATP, 10 mM β-glycerol phosphate, 1 mM sodium orthovanadate and 1 mM dithiothreitol. Proceed to KinaseGlo luciferase reaction.

Following the completion of the kinase reaction an equal volume of KinaseGlo luciferase reagent (Promega) is added and the luminescence read using a luminescence plate reader within 5-10 minutes. Compound activity is expressed as % inhibition relative to maximal inhibition observed at the maximal dose and IC50 values then calculated using curve fitting software (GraphPad Prizm).

### EXAMPLES

### Example 1:

### (3S)-8-amino-9-fluoro-2,3-dihydro-3-methyl-7-oxo-10-[(1S,3S)-3-(2-pyridyl)cyclope ntylamino]-7H-pyrido[1,2,3-de][1,4]benzoxazine-6-carbonitrile

### Step 1: 3-(2-pyridyl)-2-cyclopenten-1-one

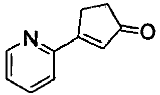

To a solution of 2-Bromopyridine (5.00g, 31.6mmol) in THF (30ml) was added dropwidse n-BuLi (2.55M in Hexane, 12.5mL. 31.9mmol) at -78 °C, and stirred for 10 min. To a reaction mixture was added dropwise 3-ethoxy-2-cyclopentenone (2.00g, 15.9mmol) in THF (10 mL) at -78 °C, and stirred at 0 °C for 2 h. The reaction mixture was acidified with 2M HCl (10 mL), and aqueous layer was washed with EtOAc. The aqueous layer was basified with 1 M NaOH aq., and then extracted with CH₂Cl₂. The organic layer was washed with brine, and dried over anhyd Na₂SO₄ followed by the removal of solvent. The crude product was purified by column chromatography (Hexane/EtOAc = 1/3) to yield 3-(2-pyridyl)-2-cyclopenten-1-one (1.57 g, 58%) as brown solid.
¹H-NMR(CDCl₃-d, 400 MHz) δ 2.60 - 2.65 (2H, m), 3.16 (2H, dt, J = 7.9, 1.8 Hz), 6.82 (1H, t, J = 1.8 Hz), 7.32 - 7.37 (1H, m), 7.70 (1H, d, J = 7.9 Hz), 7.79 (1H, td, J = 7.9,1.8 Hz), 8.73 (1H, d, J = 4.9 Hz).
EIMS (+) 159 [M] ⁺.

### Step 2: 3-(2-pyridyl)cyclopentan-1-one

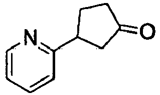

A mixture of 3-(2-pyridyl)-2-cyclopenten-1-one (1.43g, 8.98mmol) and 10% Pd/C (142mg) in EtOH (30mL) was stirred under hydrogen atmosphere (0.3 MPa) for 6.5 h. The catalyst was removed by filtration over Celite and the filtrate was concentrated in vacuo. The crude product was purified by column chromatography (Hexane/EtOAc = 1/1) to yield 3-(2-pyridyl)cyclopentan-1-one (3.26 g, 94%) as colorless oil.
1H-NMR(CDCl₃-d, 400 MHz) δ 2.13 - 2.24 (1H, m), 2.26 - 2.35 (1H, m), 2.36 - 2.55 (2H, m), 2.56 - 2.75 (2H, m), 3.56 (1H, qui, J = 6.7 Hz), 7.10 - 7.23 (2H, m), 7.64 (1 H, td, J = 7.3, 1.2 Hz), 8.57 (1H, d, J = 4.2 Hz).
EIMS (+) 161 [M] +.

### Step 3: cis-3-(2-pyridyl)cyclopentanol

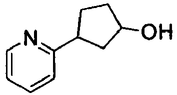

To a solution of 3-(2-pyridyl)cyclopentan-1-one (3.17g, 19.7mmol) in MeOH (80ml) was added portionwidse NaBH4 (745mg, 19.7mmol) at 0 °C, and stirred for 2 h at room temperature. The reaction mixture was poured into ice-water and then extracted with EtOAc. The organic layer was dried over anhyd Na₂SO₄ followed by the removal of solvent. The crude product was purified by column chromatography (Hexane/EtOAc = 1/1 → 1/3) to yield cis-3-(2-pyridyl)cyclopentanol (1.90g, 59%) as colorless oil.
1H-NMR(CDCl₃-d, 400 MHz) δ 1.70 - 2.00 (4H, m), 2.12 - 2.30 (2H, m), 3.42 (1H, dd, J =15.2, 9.7 Hz), 4.35 (1H, t, J = 4.3 Hz), 6.47(1H, brs), 7.10 (1H, dd, J = 7.3, 5.5 Hz), 7.17 (1H, d, J = 7.3 Hz), 7.59 (1H, t, J = 7.3 Hz), 8.51 (1H, d, J = 4.3 Hz).
EIMS (+) 163 [M] +.

### Step 4: trans-N-[3-(2-pyridyl)cyclopentyl]phthalimide

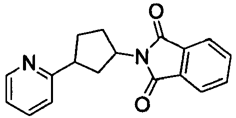

A solution of cis-3-(2-pyridyl)cyclopentanol (1.86g, 11.4mmol) in THF (100mL) was cooled to 0 °C and treated with phthalimide (1.85g, 12.5mmol), diethylazodicarboxylate (2.2M in toluene, 5.7mL, 11.4mmol) and triphenylphosphine (3.29g, 12.5mmol). The reaction mixture was stirred at room temperature for overnight. The solvent was removed, and the crude product was purified by column chromatography (Hexane/EtOAc = 3/1 → 2/1 → 1/1) to yield trans-N-[3-(2-pyridyl)cyclopentyl]phthalimide (1.52g, 41%) as a colorless powder.
¹H-NMR(CDCl₃-d, 400 MHz) δ 1.87-2.02 (1H, m), 2.15-2.41 (4H, m), 2.47 (1H, ddd, J = 13.4, 8.6, 6.1 Hz), 3.81(1H, qui, J = 8.6 Hz), 4.92 - 5.03 (1H, m), 7.11(1H, dd, J = 7.3, 5.2 Hz), 7.22 (1H, d, J = 7.3 Hz), 7.60 (1H, td, J = 7.3,1.2 Hz), 7.69 -7.73 (2H, m), 7.81 - 7.85 (2H, m), 8.5.7 (1H, d, J = 4.3 Hz).
EIMS (+) 292 [M] ⁺.

### Step 5:

### (1S,3S)-N-[3-(2-pyridyl)cyclopentyl]phthalimide and (1R,3R)-N-[3-(2-pyridyl)cyclopentyl]phthalimide

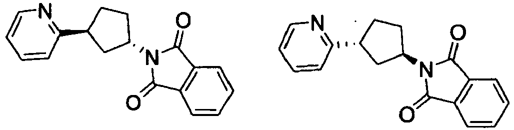

Trans-N-[3-(2-pyridyl)cyclopentyl]phthalimide (10.0g) was separated by preparative HPLC using a Chiralpak IA column (Φ20 x 250 mm) and MeCN-CH₂Cl₂ (9/1) as the eluent at a flow rate 5 mL/min for 1h. The UV detector was set at 300 nm, the injection loop volume was 5 mL, and the injection load was 68-70 mg in a MeCN-CH₂Cl₂ (9/1) solution.
(1S,3S)-N-[3-(2-pyridyl)cyclopentyl]phthalimide; 4.78 g, >99% purify with >99% e.e. (Chiralpak IA column (Φ4.6 x 250 mm), MeCN-CH₂Cl₂ (9/1), 0.5 mL/min, Rt=10.3 min)
(1R,3R)-N-[3-(2-pyridyl)cyclopentyl]phthalimide; 4.85 g, >99% purify with >99% e.e. (Chiralpak IA column (Φ4.6 x 250 mm), MeCN-CH₂Cl₂ (9/1), 0.5 mL/min, Rt=13.4 min)

### Step 6: (1S, 3S)-3-(2-pyridyl)cyclopentylamine

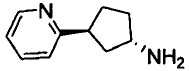

A mixture of (1S,3S)-N-[3-(2-pyridyl)cyclopentyl]phthalimide (4.12g, 14.1mmol), and hydrazinemonohydorate (2.0mL, 41.2mmol) in EtOH (100 mL) was stirred at reflux for 3 h. After cooling, the reaction mixture was filtered followed by the removal of solvent. The residure was suspended in EtOAc and stirred at reflux for 1 h. After cooling, the precipitate was filtered followed by the removal of solvent to yield (1S, 3S)-3-(2-pyridyl)cyclopentylamine (2.10 g, 92%) as yellow oil.
¹H-NMR(CDCl₃-d, 400 MHz) δ 1.40 -1.63 (3H, m), 1.77 - 1.92 (2H, m), 2.10 - 2.28 (3H, m), 3.49 (1 H, qui, J = 8.6 Hz), 3.66 (1H, qui, J = 6.1 Hz), 7.08(1H, dd, J = 7.9, 5.5 Hz), 7.16(1H, d, J = 7.9 Hz), 7.57 (1H, td, J = 7.9, 1.2 Hz), 8.54 (1H, d, J = 4.3 Hz).
CIMS (+) 163 [M+H] ⁺.

### Step 7:

### (3S)-8-amino-9-fluoro-2,3-dihydro-3-methyl-7-oxo-10-[(1S,3S)-3-(2-pyridyl)cyclope ntylamino]-7H-pyrido[1,2,3-de][1,4] benzoxazine-6-carbonitrile

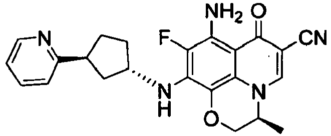

A mixture of (3S)-8-amino-9,10-difluoro-2,3-dihydro-3-methyl-7-oxo-7H-pyrido[1,2,3-de][1,4]ben zoxazine-6-carbonitrile (1.30g, 4.69mmol), (1S, 3S)-3-(2-pyridyl)cyclopentylamine (913 mg, 5.63 mmol) and triethylamine (1.30mL, 9.33mmol) in anhydrous DMSO (8 mL) was stirred at 100°C for 7 h. After cooling, the reaction mixture was poured into ice-water and then extracted with CH₂Cl₂-MeOH. The organic layer was washed with water and brine, and dried over anhyd Na₂SO₄ followed by the removal of solvent. The crude product was purified by column chromatography (EtOAc) to yield (3S)-8-amino-9-fluoro-2,3-dihydro-3-methyl-7-oxo-10-[(S,3S)-3-(2-pyridyl)cyclope ntylamino]-7H-pyrido[1,2,3-de][1,4]benzoxazine-6-carbonitrile (364mg, 19%) as a yellow powder.
¹H-NMR(DMSO-d₆, 400 MHz) δ 1.35 (3H, d, J = 6.7 Hz), 1.66-1.82 (2H, m), 1.96 -2.06 (1H, m), 2.06 - 2.20 (3H, m), 3.43 (1H, t, J = 8.3 Hz), 4.05 (1H, dd, J = 11.0, 1.8 Hz), 4.32 (1H, dd, J = 11.0, 1.8 Hz), 4.39 - 4.52 (2H, m), 5.17 (1 H, dd, J = 7.9, 1.8 Hz), 6.94 (2H, brs), 7.17 (1H, dd, J = 7.9, 4.9 Hz), 7.26 (1H, d, J = 7.9 Hz), 7.67 (1H, td, J = 7.9, 1.8 Hz), 8.46 (1H, s), 8.48 (1H, d, J = 4.3 Hz).
ESIMS (+) 420 [M+H] ⁺.
HRESIMS (+) 420.18345 (Calcd for C₂₃H₂₃FN₅O₂, 420.18358).
anal. C 65.57% H 5.24% N 16.31%, Calcd for C₂₃H₂₂FN₅O₂, 0.2H₂O, C 65.30% H 5.34% N 16.55%.

### Example 2:

### (3S)-8-amino-9-fluoro-2,3-dihydro-3-methyl-7-oxo-10-[(1R,3R)-3-(2-pyridyl)cyclope ntylamino]-7H-pyrido[1,2,3-de][1,4]benzoxazine-6-carbonitrile

### Step 1: (1R, 3R)-3-(2-pyridyl)cyclopentylamine

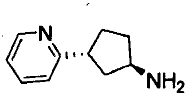

A mixture of (1R,3R)-N-[3-(2-pyridyl)cyclopentyl]phthalimide (4.17g, 14.3mmol), and hydrazinemonohydorate (2.0mL, 41.2mmol) in EtOH (100mL) was stirred at reflux for 4 h. After cooling, the reaction mixture was filtered followed by the removal of solvent. The residue was suspended in EtOAc and stirred at reflux for 1 h. After cooling, the precipitate was filtered followed by the removal of solvent to yield (1R, 3R)-3-(2-pyridyl)cyclopentylamine (1.62 g, 70%) as yellow oil.
¹H-NMR(CDCl₃-d, 400 MHz) δ 1.40 -1.63 (3H, m), 1.77 - 1.92 (2H, m), 2.10 - 2.28 (3H, m), 3.49 (1H, qui, J = 8.6 Hz), 3.66 (1 H, qui, J = 6.1 Hz), 7.08(1H, dd, J = 7.9, 5.5 Hz), 7.16(1H, d, J = 7.9 Hz), 7.57 (1 H, td, J = 7.9, 1.2 Hz), 8.54 (1H, d, J = 4.3 Hz).
CIMS (+) 163 [M+H] ⁺.

### Step 2:

### (3S)-8-amino-9-fluoro-2,3-dihydro-3-methyl-7-oxo-10-[(1R,3R)-3-(2-pyridyl)cyclope ntylamino]-7H-pyrido[1,2,3-de][1,4]benzoxazine-6-carbonitrile

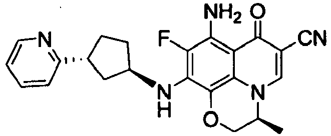

A mixture of (3S)-8-amino-9,10-difluoro-2,3-dihydro-3-methyl-7-oxo-7H-pyrido[1,2,3-de][1,4]ben zoxazine-6-carbonitrile (1.40g, 5.05mmol), (1R, 3R)-3-(2-pyridyl)cyclopentylamine (985mg, 6.07 mmol) and triethylamine (1.40mL, 10.0mmol) in anhydrous DMSO (8mL) was stirred at 100°C for 7 h. After cooling, the reaction mixture was poured into ice-water and then extracted with CH₂Cl₂-MeOH. The organic layer was washed with water and brine, and dried over anhyd Na₂SO₄ followed by the removal of solvent. The crude product was purified by column chromatography (EtOAc) to yield (3S)-8-amino-9-fluoro-2,3-dihydro-3-methyl-7-oxo-10-[(1R,3R)-3-(2-pyridyl)cyclope ntylamino]-7H-pyrido[1,2,3-de][1,4]benzoxazine-6-carbonitrile (325mg, 15%) as a yellow powder.
¹H-NMR(DMSO-d₆, 400 MHz) δ 1.35 (3H, d, J = 6.7 Hz), 1.66-1.82 (2H, m), 1.95 -2.04 (1H, m), 2.05 - 2.23 (3H, m), 3.42 (1H, qui, J = 7.9 Hz), 4.06 (1H, dd, J =11.0, 1.8 Hz), 4.33 (1H, dd, J = 11.0, 1.8 Hz), 4.40 - 4.52 (2H, m), 5.17 (1H, dd, J = 7.9, 1.8 Hz), 6.94 (2H, brs), 7.17 (1H, dd, J = 7.3, 4.9 Hz), 7.26 (1H, d, J = 7.9 Hz), 7.67 (1H, td, J = 7.3, 1.8 Hz), 8.46 (1H, s), 8.48 (1H, d, J = 4.3 Hz).
ESIMS (+) 420 [M+H] ⁺.
HRESIMS (+) 420.18368 (Calcd for C₂₃H₂₃FN₅O₂, 420.18358).
anal. C 65.61% H 5.28% N 16.19%, Calcd for C₂₃H₂₂FN₅O₂, C 65.86% H 5.29% N 16.70%.

### Example 3:

### (3S)-8-amino-9-fluoro-2,3-dihydro-3-methyl-7-oxo-10-[cis-3-(2-pyridyl)cyclopentyla mino]-7H-pyrido[1,2,3-de][1,4]benzoxazine-6-carbonitrile

### Step 1: trans-3-(2-pyridyl)cyclopentanol

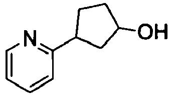

To a solution of 3-(2-pyridyl)cyclopentan-1-one (3.17 g, 19.7 mmol) in MeOH (80 ml) was added portionwidse NaBH₄ (745 mg. 19.7 mmol) at 0°C, and stirred for 2 h at room temperature. The reaction mixture was poured into ice-water and then extracted with EtOAc. The organic layer was dried over anhyd Na₂SO₄ followed by the removal of solvent. The crude product was purified by column chromatography (Hexane / EtOAc = 1 / 1 → 1 / 3) to yield trans-3-(2-pyridyl)cyclopentanol (965 mg, 30%) as colorless oil.
¹H-NMR(CDCl₃-d, 400 MHz) δ 1.50 -1.78 (2H, m), 1.80 - 1.93 (1H, m), 2.04 - 2.13 (2H, m), 2.16 - 2.33 (2H, m), 3.55 (1H, qui, J = 7.9 Hz), 4.52 - 4.62 (1H, m), 7.09 (1H, dd, J = 7.3, 5.5 Hz), 7.17 (1H, d, J = 7.3 Hz), 7.58 (1H, td, J = 7.3, 1.8 Hz), 8.54 (1H, d, J = 4.3 Hz).
EIMS (+) 163 [M]⁺.

### Step 2: cis-N-[3-(2-pyridyl)cyclopentyl]phthalimide

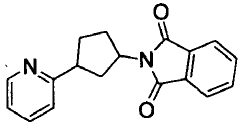

A solution of trans-3-(2-pyridyl)cyclopentanol (957mg, 5.86mmol) in THF (25 mL) was cooled to 0°C and treated with phthalimide (1.29g, 8.77mmol), cyanomethylenetri-n-butylphosphorane (2.12 g, 8.78 mmol). The reaction mixture was stirred at 70 °C for 8 h. The solvent was removed, and the crude product was purified by column chromatography (Hexane/EtOAc = 3/1 → 1/1) and triturated with Hexane-EtOAc to yield cis-N-[3-(2-pyridyl)cyclopentyl]phthalimide (901 mg, 53%) as a pale yellow powder.
¹H-NMR(CDCl₃-d, 400 MHz) δ 2.10 - 2.40 (5H, m), 2.56 (1H, dd, J = 22.6, 12.2 Hz), 3.28 - 3.40(1H, m), 4.80 - 4.90 (1H, m), 7.13(1H, dd, J = 7.9, 5.9 Hz), 7.35(1H, d, J = 7.9 Hz), 7.63 (1H, t, J = 7.9 Hz), 7.71 (2H, dd, J = 5.5, 3.0 Hz), 7.83 (2H, dd, J = 5.5, 3.0 Hz), 8.55 (1H, d, J = 4.3 Hz).
EIMS (+) 292 [M] ⁺.

### Step 3: cis-3-(2-pyridyl)cyclopentylamine

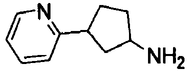

The title compound (498mg, 99%) was prepared from cis-N-[3-(2-pyridyl)cyclopentyl]phthalimide (905 mg, 3.10 mmol) in a manner similar to that described for the preparation of trans-3-(2-pyridyl)cyclopentylamine.
¹H-NMR(CDCl₃-d, 400 MHz) δ 1.52 -1.74 (3H, m), 1.95 - 2.13 (3H, m), 2.31 - 2.40 (1H, m), 3.26 (1H, qui, J = 7.9 Hz), 3.48 (1H, qui, J = 6.1 Hz), 7.09(1H, dd, J = 7.9, 4.9 Hz), 7.18 (1H, d, J = 7.9 Hz), 7.58 (1H, td, J = 7.9, 1.2 Hz), 8.54 (1H, d, J = 4.3 Hz).
CIMS (+) 163 [M+H] ⁺.

### Step 4:

### (3S)-8-amino-9-fluoro-2,3-dihydro-3-methyl-7-oxo-10-[cis-3-(2-pyridyl)cyclopentyla mino]-7H-pyrido[1,2,3-de][1,4]benzoxazine-6-carbonitrile

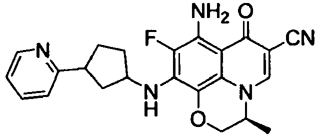

A mixture of (3S)-8-amino-9,10-difluoro-2,3-dihydro-3-methyl-7-oxo-7H-pyrido[1,2,3-de][1,4]ben zoxazine-6-carbonitrile (180 mg, 0.649 mmol), cis-3-(2-pyridyl)cyclopentylamine (158 mg, 0.973 mmol) and triethylamine (0.200 mL, 0.976 mmol) in anhydrous DMSO (3 mL) was stirred at 80 °C for 8 h. After cooling, the reaction mixture was poured into ice-water and then extracted with CH₂Cl₂-MeOH. The organic layer was washed with water and brine, and dried over anhyd Na₂SO₄ followed by the removal of solvent. The crude product was purified by column chromatography (Hexane/EtOAc = 1/3) to yield
(3S)-8-amino-9-fluoro-2,3-dihydro-3-methyl-7-oxo-10-[cis-3-(2-pyridyl)cyclopentyla mino]-7H-pyrido[1,2,3-de][1,4]benzoxazine-6-carbonitrile (diastereomer mixture, 55.6 mg, 20%) as a yellow powder.
¹H-NMR(DMSO-d₆, 400 MHz) δ1.34 (3H×1/2, d, J = 6.7 Hz), 1.37 (3H×1/2, d, J = 6.7 Hz), 1.65 - 1.93 (5H, m), 2.03 - 2.12 (1H, m), 2.27 - 2.37 (2H, m), 3.34 - 3.40 (1H, m), 3.99-4.10 (1H, m), 4.36 (1H, dd J = 11.6. 3.7 Hz), 4.40-4.49 (2H, m), 6.56 (1H×1/2, d, J = 7.3 Hz), 6.70 (1H×1/2, d, J = 7.3 Hz), 6.93 (2H, brs), 7.22 (1H, t, J = 6.1 Hz), 7.30 (1H, d, J = 7.3 Hz), 7.70 (1H, t, J = 7.3 Hz), 8.45 (1H, s), 8.55 (1H, t, J = 5.5 Hz).
ESIMS (+) 420[M+H] ⁺.
HRESIMS (+) 420.18374 (Calcd for C₂₃H₂₃FN₅O₂, 420.18358).

### Example 4:

### (3S)-8-amino-9-fluoro-2,3-dihydro-3-methyl-7-oxo-10-[trans-3-(1H-pyrazol-1-yl)cyc lopentylamino]-7H-pyrido[1,2,3-de][1,4]benzoxazine-6-carbonitrile (F)

### Step 1:

### 3-(1H-pyrazol-1-yl)cyclopentan-1-one

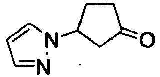

A mixture of pyrazole (6.85g, 100mmol), 2-cyclopenten-1-one (19.3g, 235mmol) and ScCl₃ (1.42g, 9.38mmol) in anhydrous CH₂Cl₂ (3mL) was stirred at room temperature for 30 min. The reaction mixture was poured into water and then extracted with CH₂Cl₂. The organic layer was washed with brine, and dried over anhyd Na₂SO₄ followed by the removal of solvent. The crude product was purified by column chromatography (Hexane / EtOAc = 4 / 1) to yield 3-(1H-pyrazol-1-yl)cyclopentan-1-one (14.1 g, 94%) as yellow oil.
¹H-NMR(CDCl₃-d, 400 MHz) δ 2.27 -2.38 (1H, m), 2.39 - 2.67 (3H, m), 2.75 (1H, dd, J = 18.3, 7.3 Hz), 2.84 (1H, dd, J =18.3, 7.3 Hz), 4.97 (1H, qui, J = 6.1 Hz), 6.28 (1H, t, J = 7.9, 1.8 Hz), 7.45 (1H, d, J = 1.8 Hz), 7.54 (1H, s).

### Step 2:

### cis-3-(1H-pyrazol-1-yl)cyclopentanol

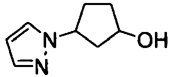

To a solution of 3-(1H-pyrazol-1-yl)cyclopentan-1-one (14.09g, 93.8mmol) in MeOH (150ml) was added portionwidse NaBH₄ (1.77g. 46.8 mmol) at 0 °C, and stirred for 30 min at room temperature. The reaction mixture was poured into 0.5M HCl and basified with sat. NaHCO₃ aq. and then extracted with CH₂Cl₂. The organic layer was dried over anhyd Na₂SO₄ followed by the removal of solvent. The crude product was purified by column chromatography (Hexane/EtOAc = 2/1 → 1/1) to yield cis-3-(1H-pyrazol-1-yl)cyclopentanol (10.41 g, 73%) as colorless oil.
¹H-NMR(CDCl₃-d, 400 MHz) δ 1.80 - 1.91 (1H, m), 1.96 - 2.19 (3H, m), 2.19 - 2.33 (2H, m), 4.35 - 4.42 (1H, m), 4.80 (1H, td, J = 7.9, 4.3 Hz), 5.75 (1H, d, J = 9.8 Hz), 6.20 (1H, t, J = 1.8 Hz), 7.43 (1H, d, J = 1.8 Hz), 7.51 (1H, s).
EIMS (+) 219 [M] ⁺.

### Step 3:

### trans-N-[3-(1H-pyrazol-1-yl)cyclopentyl]phthalimide

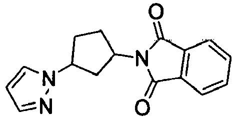

A solution of cis-3-(1H-pyrazol-1-yl)cyclopentanol (5.01g, 32.9mmol) in THF (150mL) was cooled to 0°C and treated with phthalimide (5.33g, 36.2mmol), diisopropylazodicarboxylate (7.1mL, 36.1mmol) and triphenylphosphine (9.48 g, 36.1 mmol). The reaction mixture was stirred at 0 °C for 4 h. The reaction mixture was poured water and then extracted with EtOAc. The organic layer was dried over anhyd Na₂SO₄ followed by the removal of solvent. The crude product was purified by column chromatography (Chromatrex, Hexane/EtOAc = 5/1) to yield trans-N-[3-(1H-pyrazol-1-yl)cyclopentyl]phthalimide (4.85g, 52%) as a colorless powder.
¹H-NMR(CDCl₃-d, 400 MHz) δ 2.16 - 2.33 (3H, m), 2.45 - 2.56 (2H, m), 2.56 - 2.65 (1H, m), 5.00 - 5.10 (1H, m), 5.16 (1H, qui, J = 7.3 Hz), 6.25 (1H, t, J = 1.8 Hz), 7.47 (1H, d, J = 1.8 Hz), 7.55 (1H, d, J = 1.8 Hz), 7.72 (2H, dd, J = 5.5, 3.1 Hz), 7.84 (2H, dd, J = 5.5, 3.1 Hz).

### Step 4:

### trans-N-[3-(1H-pyrazol-1-yl)cyclopentyl]phthalimide (F) and trans-N-[3-(1H-pyrazol-1-yl)cyclopentyl]phthalimide (R)

Trans-N-[3-(1H-pyrazol-1-yl)cyclopentyl]phthalimide (3.12g) was separated by preparative HPLC using a Chiralpak IA column (Φ20 x 250mm) and MeCN as the eluent at a flow rate 5mL/min for 1h. The UV detector was set at 300 nm, the injection loop volume was 5mL, and the injection load was 80 mg in a MeCN solution. trans-N-[3-(1H-pyrazol-1-yl)cyclopentyl]phthalimide (F); 1.56g, >99% purify with >99% e.e. (Chiralpak IA column (Φ4.6 x 250 mm), MeCN, 0.5 mL/min, Rt=10.3 min) trans-N-[3-(1H-pyrazol-1-yl)cyclopentyl]phthalimide (R); 1.5 g, >99% purify with >99% e.e. (Chiralpak IA column (Φ4.6 x 250 mm), MeCN, 0.5 mL/min, Rt=15.2 min)

### Step 5: trans-3-(1H-pyrazol-1-yl)cyclopentylamine (F)

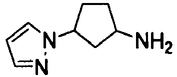

A mixture of trans-N-[3-(1H-pyrazol-1-yl)cyclopentyl]phthalimide (F) (1.53g, 5.44mmol), and hydrazinemonohydorate (0.53mL, 10.9mmol) in EtOH (30 mL) was stirred at reflux for 4 h. After cooling, the reaction mixture was filtered followed by the removal of solvent. The crude product was purified by column chromatography (Chromatrex, CH₂Cl₂/EtOH = 30/1) to yield trans-3-(1H-pyrazol-1-yl)cyclopentylamine (427mg, 52%) as yellow oil.
¹H-NMR(CDCl₃-d, 400 MHz) δ 1.30 -1.50 (3H, m), 1.88 - 1.98 (1H, m), 2.01 - 2.10 (1H, m), 2.15 - 2.25 (1H, m), 2.27 - 2.42 (2H, m), 3.74 (1H, qui, J = 6.1 Hz), 4.88 (1H, qui, J = 7.3 Hz), 6.22 (1H, d, J = 1.8 Hz), 7.40 (1H, d, J = 1.8 Hz), 7.51 (1H, s).

### Step 6:

### (3S)-8-amino-9-fluoro-2,3-dihydro-3-methyl-7-oxo-10-[trans-3-(1H-pyrazol-1-yl)cyc lopentylamino]-7H-pyrido[1,2,3-de][1,4]benzoxazine-6-carbonitrile (F)

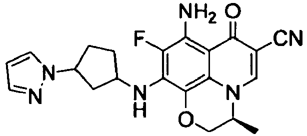

A mixture of (3S)-8-amino-9,10-difluoro-2,3-dihydro-3-methyl-7-oxo-7H-pyrido[1,2,3-de][1,4]ben zoxazine-6-carbonitrile (580mg, 2.09mmol), trans-3-(1H-pyrazol-1-yl)cyclopentylamine (380mg, 2.51mmol) and di-isopropylethylamine (0.80mL, 4.59mmol) in anhydrous DMSO (12mL) was stirred at 100 °C for 7 h. After cooling, the reaction mixture was poured into ice-water and then extracted with CH₂Cl₂-MeOH. The organic layer was washed with water and brine, and dried over anhyd Na₂SO₄ followed by the removal of solvent. The crude product was purified by column chromatography (EtOAc) to yield (3S)-8-amino-9-fluoro-2,3-dihydro-3-methyl-7-oxo-10-[trans-3-(1H-pyrazol-1-yl)cyc lopentylamino]-7H-pyrido[1,2,3-de][1,4]benzoxazine-6-carbonitrile (125 mg, 15%) as a yellow powder.
¹H-NMR(DMSO-d₆, 400 MHz) δ 1.35 (3H, d, J = 6.7 Hz), 1.60 -1.73 (1H, m), 1.86-1.98 (1H, m), 2.11 - 2.29 (4H, m), 4.04 (1H, d, J = 10.3 Hz), 4.31 (1H, d, J = 10.3 Hz), 4.44 (1H, q, J= 6.7 Hz), 4.55 (1H, q, J = 6.7 Hz), 4.89 (1H, qui, J = 6.7 Hz), 5.31 (1H, d, J = 8.5 Hz), 6.20 (1 H, t, J = 1.8 Hz), 6.95 (2H, brs), 7.43 (1H, s), 7.74 (1H, d, J = 1.8 Hz), 8.46 (1H, s).
ESIMS (+) 409 [M+H] ⁺.
HRESIMS (+) 409.17920 (Calcd for C₂₁H₂₂FN₆O₂, 409.17883).
anal. C 61.70%, H 5.17%, N 20.25%, Calcd for C₂₁H₂₁FN₆O₂, 0.1H₂O, C 61.48%, H 5.21%, N 20.49%.

### Example 5:

### (3S)-8-amino-9-fluoro-2,3-dihydro-3-methyl-7-oxo-10-[trans-3-(1H-pyrazol-1-yl)cyc lopentylamino]-7H-pyrido[1,2,3-de][1,4]benzoxazine-6-carbonitrile (R)

### Step 1: trans-3-(1H-pyrazol-1-yl)cyclopentylamine (R)

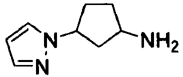

A mixture oftrans-N-[3-(1H-pyrazol-1-yl)cyclopentyl]phthalimide (R) (1.49g, 5.30mmol), and hydrazinemonohydorate (0.51mL, 10.5mmol) in EtOH (30mL) was stirred at reflux for 3h. After cooling, the reaction mixture was filtered followed by the removal of solvent. The crude product was purified by column chromatography (Chromatrex, CH₂Cl₂/EtOH = 30/1) to yield trans-3-(1H-pyrazol-1-yl)cyclopentylamine (517 mg, 65%) as yellow oil.
¹H-NMR(CDCl₃-d, 400 MHz) δ 1.30 -1.50 (3H, m), 1.88 - 1.98 (1H, m), 2.01 - 2.10 (1H,m),2.15-2.25 (1H, m), 2.27 - 2.42 (2H, m), 3.74 (1H, qui, J = 6.1 Hz), 4.88 (1H, qui, J = 7.3 Hz), 6.22 (1H, d, J = 1.8 Hz), 7.40 (1H, d, J = 1.8 Hz), 7.51 (1H, s).
CIMS (+) 152 [M+H] ⁺.

### Step 2:

### (3S)-8-amino-9-fluoro-2,3-dihydro-3-methyl-7-oxo-10-[trans-3-(1H-pyrazol-1-yl)cyc lopentylamino]-7H-pyrido[1,2,3-de][1,4]benzoxazine-6-carbonitrile (R)

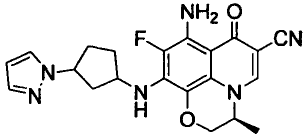

A mixture of (3S)-8-amino-9,10-difluoro-2,3-dihydro-3-methyl-7-oxo-7H-pyrido[1,2,3-de][1,4]ben zoxazine-6-carbonitrile (702mg, 2.53mmol), trans-3-(1H-pyrazol-1-yl)cyclopentylamine (460mg, 3.04mmol) and di-isopropylethylamine (0.970mL, 5.57mmol) in anhydrous DMSO (14mL) was stirred at 100 °C for 7h. After cooling, the reaction mixture was poured into ice-water and then extracted with CH₂Cl₂-MeOH. The organic layer was washed with water and brine, and dried over anhyd Na₂SO₄ followed by the removal of solvent. The crude product was purified by column chromatography (Hexane/EtOAc = 1 /10 → EtOAc) to yield
(3S)-8-amino-9-fluoro-2,3-dihydro-3-methyl-7-oxo-10-[trans-3-(1H-pyrazol-1-yl)cyc lopentylamino]-7H-pyrido[1,2,3-de][1,4]benzoxazine-6-carbonitrile (108mg, 10%) as a yellow powder.
¹H-NMR(DMSO-d₆, 400 MHz) δ 1.35 (3H, d, J = 6.7 Hz), 1.63 -1.75 (1H, m), 1.86-1.98 (1H, m), 2.07 - 2.30 (4H, m), 4.05 (1H, d, J = 11.5 Hz), 4.31 (1 H, d, J = 10.9 Hz), 4.44 (1H, q, J = 6.7 Hz), 4.54 (1H, q, J = 6.7 Hz), 4.89 (1H, qui, J = 6.7 Hz), 5.30 (1H, d, J = 8.5 Hz), 6.20 (1H, t, J = 1.8 Hz), 6.94 (2H, brs), 7.42 (1H, s), 7.74 (1H, d, J = 1.8 Hz), 8.46 (1H, s).
ESIMS (+) 409 [M+H] ⁺.
HRESIMS (+) 409.17826 (Calcd for C₂₁H₂₂FN₆O₂, 409.17883).
anal. C 61.16% H 5.08% N 20.06%, Calcd for C₂₁H₂₁FN₆O₂, 0.3H₂O, C 60.95% H 5.26% N 20.31%.

### Example 6:

### (3S)-8-amino-9-fluoro-2,3-dihydro-3-methyl-7-oxo-10-[cis-4-(3-pyridyl)cyclohexyla mino]-7H-pyrido[1,2,3-de][1,4]benzoxazine-6-carbonitrile

### Step 1 : 4-hydroxy-4-(3-pyridyl)cyclohexanone ethylene acetal

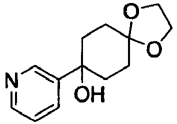

To a solution of n-BuLi (1.59 M in Hexane, 45mL. 71.6mmol) in dry ether (150mL) was added a solution of 3-Bromopyridine (11.24 g, 71.1mmol) in dry ether (70mL) dropwidse at -78°C. After stirring for 10min, a solution of cyclohexane-1,4-dione monoetylene acetal (9.25g, 59.2mmol) in THF (70mL) was added over a period of 20min at -78 °C, and stirred for 3 h. To the reaction mixture was added with water. The aqueous layer was extracted with CH₂Cl₂, and the organic layer was washed with brine. The extracts were combined with the organic layer. After drying and concentration, the crude product was purified by column chromatography (Hexane/EtOAc = 2/1) to yield 4-hydroxy-4-(3-pyridyl)cyclohexanone ethylene acetal (6.55g, 47%) as a pale yellow powder.
¹H-NMR(CDCl₃-d, 400 MHz) δ 1.65 - 1.78 (3H, m), 1.84 (2H, d, J = 11.6 Hz), 2.05-2.23 (4H, m), 3.96 - 4.05 (4H, m), 7.27 (1H, dd, J = 7.9, 4.8 Hz), 7.84 (1H, dt, J = 7.9, 1.2 Hz), 8.49 (1H, dd, J = 4.9.1.2 Hz), 8.78 (1H, d, J = 1.2 Hz).
EIMS (+) 235 [M] ⁺.

### Step 2 : 4-(3-pyridyl)cyclohex-3-en-1-one ethylene acetal

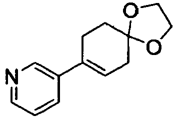

Thionyl chloride (10 mL) was added to a solution of 4-hydroxy-4-(3-pyridyl)cyclohexanone ethylene acetal (6.53g. 27.8mmol) in pyridine (60mL) at -10 °C. After stirring 0°C for 30min, the reaction mixture was poured onto ice. The aqueous layer was adjusted to pH 8 with 2M NaOH aq., and extracted with CH₂Cl₂, After drying and concentration, the crude product was purified by column chromatography (Hexane/EtOAc = 2/1) to yield 4-(3-pyridyl)cyclohex-3-en-1-one ethylene acetal (4.85 g, 62%) as colorless oil.
¹H-NMR(CDCl₃-d, 400 MHz) δ 1.94 (2H, d, J = 6.7 Hz), 2.47 - 2.51 (2H, m), 2.64-2.70 (2H, m), 4.03 (4H, s), 6.03 - 6.07 (1H, m), 7.23 (1H, dd, J = 7.9, 5.5 Hz), 7.67 (1H, dt, J = 7.9, 1.8 Hz), 8.46 (1H, dd, J = 4.9. 1.8 Hz), 8.66 (1H, d, J = 1.8 Hz).

### Step 3: 4-(3-pyridyl)cyclohexanone ethylene acetal

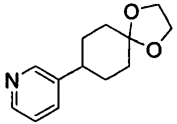

A mixture of 4-(3-pyridyl)cyclohex-3-en-1-one ethylene acetal (4.80g, 22.1mmol) in EtOAc (50mL) containing 10% Pd/C (458mg) was hydrogenated under atmospheric pressure for 3 h. After filtration of the catalyst, the solution was concentrated in vacuo, to yield 4-(3-pyridyl)cyclohexanone ethylene acetal (4.81 g, 99%) as yellow solid.
¹H-NMR(CDCl₃-d, 400 MHz)δ 1.66 -1.76 (6H, m), 1.80 (2H, d, J =11.6 Hz), 1.88 (4H, d, J = 11.0 Hz), 2.60 (1H, t, J = 11.0 Hz), 3.99 (4H, s), 7.22 (1H, dd, J = 7.9, 4.9 Hz), 7.56 (1H, d, J = 7.9 Hz), 8.44 (1H, d, J = 4.9 Hz), 8.50 (1H, d, J = 1.2 Hz).
EIMS (+) 219 [M] ⁺.

### Step 4 : 4-(3-pyridyl)cyclohexanone

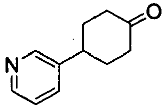

Water (1 mL) was added to a cod solution of 4-(3-pyridyl)cyclohexanone ethylene acetal (4.79g, 21.9mmol) in CF₃CO₂H (20mL). After stirring room temperature for 6 h, the reaction mixture was added dropwise to sat. NaHCO₃ aq. The resulting mixture was adjusted to pH 8 with 2M NaOH aq. and extracted with CH₂Cl₂. After drying and consentration, the crude product was purified by column chromatography (Hexane/EtOAc = 1/1 → 0/1) to yield 4-(3-pyridyl)cyclohexanone (3.23g, 84%) as a colorless powder.
¹H-NMR(CDCl₃-d, 400 MHz) δ 1.90 - 2.04 (2H, m), 2.22 - 2.30 (2H, m), 2.47 - 2.61 (4H, m), 3.07 (1H, tt, J = 12.2, 3.1 Hz), 7.23 - 7.30 (1H, m), 7.55 (1H, d, J = 7.9 Hz), 8.50 (1H, dd, J = 4.9, 1.2 Hz), 8.55 (1H, d, J = 1.2 Hz).
EIMS (+) 175 [M] ⁺.

### Step 5 : trans-4-(3-pyridyl)cyclohexanol

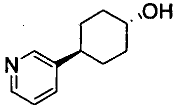

To a solution of 4-(3-pyridyl)cyclohexanone (3.22g, 18.4mmol) in MeOH (25ml) was added portionwise NaBH₄ (348mg. 9.30mmol) at 0 °C, and stirred for 30 min at room temperature. The reaction mixture was poured into 0.5M HCl and basified with sat. NaHCO₃ aq. and then extracted with CH₂Cl₂. The organic layer was dried over anhyd Na₂SO₄ followed by the removal of solvent. The crude product was purified by column chromatography (Hexane/EtOAc = 5/1) to yield trans-4-(3-pyridyl)cyclohexanol (2.48g, 76%) as a colorless powder.
¹H-NMR(CDCl₃-d, 400 MHz)δ 1.38 - 1.61 (4H, m), 1.90 - 1.98 (2H, m), 2.09 - 2.16 (2H, m), 2.53 (1H, tt, J = 11.6, 3.7 Hz), 3.65 - 3.76 (1H, m), 7.22 (1H, dd, J = 7.9, 4.9 Hz), 7.51 (1H, dt, J = 7.9, 1.8 Hz), 8.44 (1H, dd, J = 4.9, 1.8 Hz), 8.48 (1H, d, J = 1.8 Hz).
CIMS (+) 178 [M+H] ⁺.

### Step 6: cis-N-[4-(3-pyridiyl)cyclohexyl]phthalimide

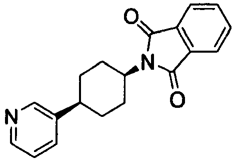

A solution of trans-4-(3-pyridyl)cyclohexanol (1.38g, 7.79mmol) in THF (70mL) was cooled to 0°C and treated with phthalimide (1.26g, 8.58mmol), diisopropylazodicarboxylate (1.7mL, 8.63mmol) and triphenylphosphine (1.26g, 8.56mmol). The reaction mixture was stirred at room temperature for 6 h. The reaction mixture was poured water and then extracted with EtOAc. The organic layer was dried over anhyd Na₂SO₄ followed by the removal of solvent. The crude product was purified by column chromatography (Hexane/EtOAc = 3/1 → 2/1) to yield cis-N-[4-(3-pyridiyl)cyclohexyl]phthalimide (1.54 g, 64%) as a colorless powder.
¹H-NMR(CDCl₃-d, 400 MHz) δ 1.65 - 1.75 (2H, m), 1.90 - 2.03 (2H, m), 2.26 - 2.48 (4H, m), 3.10 - 3.18 (1H, m), 4.25 - 4.35 (1H, m), 7.30 (1H, dd, J = 7.9, 4.9 Hz), 7.69 (2H, dd, J = 5.5, 3.0 Hz), 7.80 (2H, dd, J = 5.5, 3.0 Hz), 7.84 (1H, d, J = 7.9 Hz), 8.47 (1H, d, J = 4.9 Hz), 8.66 (1H, s).
EIMS (+) 306 [M] ⁺.

### Step 7 : cis-4-(3-pyridiyl)cyclohexylamine.

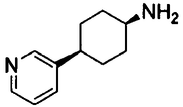

A solution of cis-N-[4-(3-pyridiyl)cyclohexyl]phthalimide (1.37g, 4.47mmol), and hydrazinemonohydorate (0.45mL, 9.00mmol) in EtOH (30mL) was stirred at reflux for 3 h. After cooling, the reaction mixture was filtered followed by the removal of solvent. The crude product was purified by column chromatography (Chromatrex, CH₂Cl₂/EtOH = 20/1) to yield cis-4-(3-pyridiyl)cyclohexylamine (705mg, 89%) as yellow oil.
¹H-NMR(CDCl₃-d, 400 MHz) δ 1.48 (2H, brs), 1.64 - 1.80 (6H, m), 1.83 - 1.95 (2H, m), 2.55 - 2.65 (1 H, m), 3.23 - 3.30 (1H, m), 7.22 (1H, dd, J = 7.9, 4.9 Hz), 7.57 (1H, d, J = 7.9 Hz), 8.43 (1H, dd, J = 4.8, 1.8 Hz), 8.51 (1H, d, J = 1.8 Hz).
EIMS (+) 176 [M]⁺.

### Step 8 :

### (3S)-8-amino-9-fluoro-2,3-dihydro-3-methyl-7-oxo-10-[cis-4-(3-pyridyl)cyclohexyla mino]-7H-pyrido[1,2,3-de][1,4]benzoxazine-6-carbonitrile

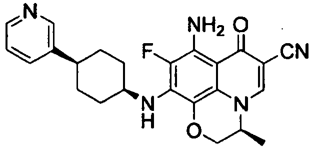

A mixture of (3S)-8-amino-9,10-difluoro-2,3-dihydro-3-methyl-7-oxo-7H-pyrido[1,2,3-de][1,4]ben zoxazine-6-carbonitrile (792mg, 2.86mmol), cis-4-(3-pyridiyl)cyclohexylamine (605 mg, 3.43 mmol) and diisopropylethylamine (1.0mL, 5.74mmol) in anhydrous DMSO (14mL) was stirred at 100°C for 8 h. After cooling, the reaction mixture was poured into ice-water and then extracted with CH₂Cl₂-MeOH. The organic layer was washed with water and brine, and dried over anhyd Na₂SO₄ followed by the removal of solvent. The crude product was purified by column chromatography (EtOAc) to yield (3S)-8-amino-9-fluoro-2,3-dihydro-3-methyl-7-oxo-10-[cis-4-(3-pyridyl)cyclohexyla mino]-7H-pyrido[1,2,3-de][1,4]benzoxazine-6-carbonitrile (152mg, 12%) as a yellow powder.
¹H-NMR(DMSO-d₆, 400 MHz) δ 1.36 (3H, d, J = 6.7 Hz), 1.59 -1.89 (8H, m), 2.62-2.71 (1H, m), 4.01 - 4.13 (2H, m), 4.40 (1 H, d, J = 11.5 Hz), 4.44 - 4.51 (1H, m), 4.92 (1H, d, J = 8.5 Hz), 6.97 (2H, brs), 7.33 (1H, dd, J = 7.9, 4.8 Hz), 7.66 (1H, d, J = 7.9 Hz), 8.40 (1H, dd, J = 4.2, 1.8 Hz), 8.47 - 8.50 (2H, m).
ESIMS (+) 434 [M+H]⁺.
HRESIMS (+) 434.20011 (Calcd for C₂₄H₂₅FN₅O₂, 434.19923).
anal. C 66.38% H 5.58% N 15.88%, Calcd for C₂₄H₂₄FN₅O₂, C 66.50% H 5.58% N 16.16%.

### Example 7:

### (3S)-8-amino-9-fluoro-2,3-dihydro-3-methyl-7-oxo-10-[trans-4-(3-pyridyl)cyclohexyl amino]-7H-pyrido[1,2,3-de][1,4]benzoxazine-6-carbonitrile

### Step 1: cis-4-(3-pyridyl)cyclohexanol

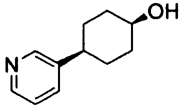

To a solution of 4-(3-pyridyl)cyclohexanone (3.22g, 18.4mmol) in MeOH (25ml) was added portionwise NaBH₄ (348mg. 9.30mmol) at 0°C, and stirred for 30min at room temperature. The reaction mixture was poured into 0.5M HCl and basified with sat. NaHCO₃ aq. and then extracted with CH₂Cl₂. The organic layer was dried over anhyd Na₂SO₄ followed by the removal of solvent. The crude product was purified by column chromatography (Hexane/EtOAc = 5/1) to yield cis-4-(3-pyridyl)cyclohexanol (510mg, 16%) as a colorless powder.
¹H-NMR(CDCl₃-d, 400 MHz) δ 1.63 -1.76 (5H, m), 1.86 - 1.98 (4H, m), 2.52 - 2.62 (1H, m), 4.13 - 4.18 (1H, m), 7.23 (1H, dd, J = 7.9, 4.3 Hz), 7.56 (1H, dt, J = 7.9, 1.8 Hz), 8.44 (1H, dd, J = 4.9, 1.8 Hz), 8.50 (1H, d, J = 2.4 Hz).
CIMS (+) 178 [M+H]⁺.

### Step 2: cis-4-(3-pyridyl)cyclohexane methanesulfanate

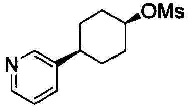

A mixture of cis-4-(3-pyridyl)cyclohexanol (302mg, 1.70mmol), methanesulfonyl chloride (0.290mL, 3.76mmol) and triethylamine (1.0mL, 3.66mmol) in anhydrous CH₂Cl₂ (15mL) was stirred at room temperature for 2.5 h. The reaction mixture was poured into ice-water and basified with sat. NaHCO₃ aq. and then extracted with CH₂Cl₂. The organic layer was dried over anhyd Na₂SO₄ followed by the removal of solvent. The crude product was purified by column chromatography (Hexane/EtOAc = 1/2) to yield cis-4-(3-pyridyl)cyclohexane methanesulfanate (423mg, 97%) as a colorless powder.
¹H-NMR(CDCl₃-d, 400 MHz) δ 1.73 -1.95 (6H, m), 2.25 (2H, d, J = 14.5 Hz), 2.58-2.68 (1H, m), 3.06 (3H, s), 5.05 - 5.12 (1H, m), 7.20 - 7.28 (1H, m), 7.55 (1H, d, J = 7.9 Hz), 8.46 (1H, dd, J = 4.8, 1.2 Hz), 8.49 (1H, d, J = 1.2 Hz).
EIMS (+) 255 [M]⁺.

### Step 3: trans-4-(3-pyridyl)cyclohexylazide

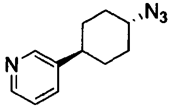

A mixture of cis-4-(3-pyridyl)cyclohexane methanesulfanate (407mg, 1.59mmol), sodium azide (507mg, 7.80mmol) in anhydrous DMF (8mL) was stirred at 60°C for 4.5 h. The reaction mixture was poured into ice-water and then extracted with CH₂Cl₂. The organic layer was dried over anhyd Na₂SO₄ followed by the removal of solvent. The crude product was purified by column chromatography (Hexane/EtOAc = 2/1) to yield trans-4-(3-pyridyl)cyclohexylazide (245mg, 76%) as colorless oil.
¹H-NMR(CDCl₃-d, 400 MHz)δ1.45 - 1.63 (4H, m), 1.95 - 2.06 (2H, m), 2.10 - 2.21 (2H, m), 2.50 - 2.61 (1H, m), 3.32 - 3.42 (1H, m), 7.22 (1H, dd J = 7.9, 4.8 Hz), 7.49 (1H, d, J = 7.9 Hz), 8.46 (1H, dd, J = 4.8, 1.8 Hz), 8.47 (1H, d, J = 1.8 Hz).
CIMS (+) 203 [M+H]

### Step 4: trans-4-(3-pyridiyl)cyclohexylamine

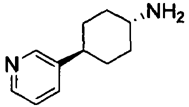

A solution of trans-4-(3-pyridyl)cyclohexylazide (238mg, 1.18mmol) in EtOH (20mL) was treated with hydrogen under atmospheric pressure over 10% Pd/C (21.2 mg) for 2 h. The catalyst was removed by filtration through Celite and the filtrate was concentrated in vacuo and dried to yield trans-4-(3-pyridiyl)cyclohexylamine (202mg, 97%) as yellow oil.
¹H-NMR(CDCl₃-d, 400 MHz) δ 1.20 -1.33 (2H, m), 1.87 - 2.04 (4H, m), 2.51 (1H, tt, J = 12.1,3.0 Hz), 2.75 (1H, tt, J =11.5, 3.0 Hz), 7.21 (1H, dd J = 7.9, 4.8 Hz), 7.51 (1H, dd, J = 7.9, 1.8 Hz), 8.44 (1H, dd, J = 4.8, 1.2 Hz), 8.48 (1H, d, J =1.2 Hz).
EIMS (+) 176 [M]⁺.

### Step 5:

### (3 S)-8-amino-9-fluoro-2,3-dihydro-3-methyl-7-oxo-10-[trans-4-(3-pyridyl)cyclohexyl amino]-7H-pyrido[1,2,3-de][1,4]benzoxazine-6-carbonitrile

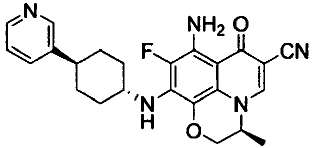

A mixture of (3S)-8-amino-9,10-difluoro-2,3-dihydro-3-methyl-7-oxo-7H-pyrido[1,2,3-de][1,4]ben zoxazine-6-carbonitrile (290mg, 1.05mmol), trans-4-(3-pyridiyl)cyclohexylamine (220mg, 1.25 mmol) and diisopropylethylamine (0.4mL, 2.30mmol) in anhydrous DMSO (4mL) was stirred at 100 °C for 8 h. After cooling, the reaction mixture was poured into ice-water and then extracted with CH₂Cl₂-MeOH. The organic layer was washed with water and brine, and dried over anhyd Na₂SO₄ followed by the removal of solvent. The crude product was purified by column chromatography (EtOAc) to yield (3S)-8-amino-9-fluoro-2,3-dihydro-3-methyl-7-oxo-10-[trans-4-(3-pyridyl)cyclohexyl amino]-7H-pyrido[1,2,3-de][1,4]benzoxazine-6-carbonitrile (33.2mg, 7%) as a yellow powder.
¹H-NMR(DMSO-d₆, 400 MHz) δ 1.35 (3H, d, J = 6.7 Hz), 1.42 -1.48 (2H, m), 1.50-1.63 (2H, m), 1.80 -1.88 (2H, m), 2.01-2.08 (2H, m), 3.70 - 3.82 (1H, m), 4.06 (1H, d, J = 11.9 Hz), 4.33 (1H, d, J = 11.9 Hz), 4.42- 4.50 (1H, m), 4.96 (1H, J = 7.9 Hz), 6.95 (2H, brs), 7.29 (1H, dd, J = 7.3, 4.8 Hz), 7.68 (1H, d, J = 7.9 Hz), 8.39 (1H, d, J = 4.8 Hz), 8.45 - 8.50 (2H, m).
ESIMS (+) 434 [M+H] ⁺.
HRESIMS (+) 434.20016 (Calcd for C₂₄H₂₅FN₅O₂, 434.19923).
anal. C 65.33% H 5.65% N 15.23%, Calcd for C₂₄H₂₄FN₅O₂, C 66.50% H 5.58% N 16.16%.

### Example 8:

### (3S)-8-amino-9-fluoro-2,3-dihydro-3-methyl-7-oxo-10-[(1S,3S)-3-(3-pyridyl)cyclope ntylamino]-7H-pyrido[1,2,3-de][1,4]benzoxazine-6-carbonitrile

### Step 1: (1S,4S)-4-(3-pyridyl)-2-cyclopenten-1-ol

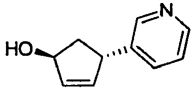

To a solution of 3-Iodopyridine (13.0g, 63.3mmol) in THF (30mL) was added i-PrMgCl (38.0mL, 76.0mmol) at 3-9 °C. After stirring was continued at room temperature for 30 min, CuCN (567mg, 6.33mmol) was added to the solution at 2 °C. After stirring was continued at 3°C for 30 min, to the resulting solution were added (1R,4S)-4-hydroxycyclopent-2-enyl acetate (3.00g, 21.1mmol) in THF (30mL). The whole mixture was stirred at the room temperature for 2 h, and diluted with saturated NH₄Cl with vigorous stirring. The products were extracted with EtOAc three times, and the combined layers were dried over Na₂SO₄. After concentration under reduced pressure, the residue was purified by column chromatography (NH, Hexane/EtOAc = 4/1) to yield (1S,4S)-4-(3-pyridyl)-2-cyclopenten-1-ol (1.88g, 55%) as yellow oil.
¹H-NMR(CDCl₃-d, 400 MHz) δ 1.81 (1H, brs), 2.04-2.14 (1H, m), 2.34 (1H, ddd, J = 14.2, 8.1, 2.6 Hz), 4.14-4.20 (1H, m), 5.08 (1H, brs), 6.03 (1H, dd, J = 5.5, 1.2 Hz), 6.07-6.12 (1H, m), 7.22 (1H, dd, J = 8.3,5.2 Hz), 7.42 (1H, dt, J = 7.9, 1.8 Hz), 7.43 (1H, d, J = 2.4 Hz), 8.46 (1H, dd, J = 4.3, 1.2 Hz).
EIMS (+) : 161.1 [M]⁺.

### Step 2: (1R,3R)-3-(3-pyridyl)cyclopentanol

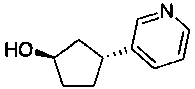

A mixture of (1S,4S)-4-(3-pyridyl)-2-cyclopenten-1-ol (1.86g, 11.5mmol) and 10% Pd/C (186 mg) in EtOH (35mL) was stirred under hydrogen atmosphere for 3 h. The catalyst was removed by filtration over Celite and the filtrate was concentrated in vacuo. (1R,3R)-3-(3-pyridyl)cyclopentanol (1.82g, 97%) was yield as yellow oil.
¹H-NMR(CDCl₃-d, 400 MHz) δ 1.56-1.88 (4H, m), 2.08-2.36 (4H, m), 3.37-3.49 (1H, m), 4.53-4.59 (1H, m), 7.24-7.30 (1H, m), 7.58 (1H, dt, J = 7.9, 1.8 Hz), 8.46 (1H, d, J = 4.3 Hz), 8.53 (1H, s).
CIMS (+) : 164.1 [M+H]⁺.

### Step 3: (1S,3R)-3-(3-pyridyl)cyclopentyl acetate

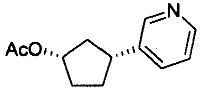

A solution of (1R,3R)-3-(3-pyridyl)cyclopentanol (3.47g, 21.3mmol) in THF (100mL) was cooled to 4°C and treated with acetic acid (1.34mL, 23.4mmol), Diisopropylazodicarboxylate (4.61mL, 23.4mmol) and triphenylphosphine (6.14 g, 23.4 mmol). The reaction mixture was stirred at room temperature for 12 h. The reaction mixture was diluted with EtOAc, and washed with H₂O and brine. The organic layers were dried over Na₂SO₄. After concentration under reduced pressure, the residue was purified by column chromatography (NH, Hexane/EtOAc = 6/1) to yield (1S,3R)-3-(3-pyridyl)cyclopentyl acetate (4.39 g, 100%) as colorless oil.
¹H-NMR(CDCl₃-d, 400 MHz) δ 1.70-2.18 (8H, m), 2.55-2.65 (1H, m), 3.03-3.15 (1H, m), 5.23-5.31 (1H, m), 7.24 (1H, dd, J = 7.9, 4.9 Hz), 7.58 (1H, dt, J = 7.9, 1.8 Hz), 8.46 (1H, dd, J=4.9, 1.8 Hz), 8.53 (1H, d, J = 2.4 Hz).
CIMS (+) : 206.1 [M+H]⁺.

### Step 4: (1S,3R)-3-(3-pyridyl)cyclopentanol

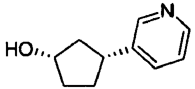

To a solution of (1S,3R)-3-(3-pyridyl)cyclopentyl acetate (5.43g, 26.5mmol) in MeOH (80ml) was added 1N NaOH aq. (26mL) at 4 °C, and stirred for 2 h at same temperature. To the reaction mixture was added H₂O and then extracted with CH₂Cl₂ four times, and the combined layers were dried over Na₂SO₄. After concentration under reduced pressure, the residue was purified by column chromatography (EtOAc) to yield (1S,3R)-3-(3-pyridyl)cyclopentanol (3.99 g, 92%) as colorless oil.
¹H-NMR(CDCl₃-d, 400 MHz) δ 1.60-2.18 (6H, m), 2.44-2.54 (1H, m), 3.02-3.13 (1H, m), 4.45-4.54 (1H, m), 7.23 (1H, dd, J = 7.3, 4.9 Hz), 7.65 (1H, d, J = 7.9 Hz), 8.43 (1H, dd, J = 4.9, 1.2 Hz), 8.52 (1H, d, J = 1.8 Hz).
CIMS (+) : 164.1 [M+H]⁺.

### Step 5: (1R,3R)-N-[3-(3-pyridyl)cyclopentyl]phthalimide

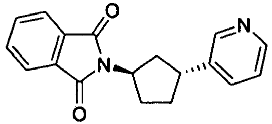

A solution of (1S,3R)-3-(3-pyridyl)cyclopentanol (3.95g, 24.2mmol) in THF (80mL) was cooled to 4 °C and treated with phthalimide (3.91g, 26.6mmol), Diisopropylazodicarboxylate (5.24mL, 26.6mmol) and triphenylphosphine (6.98 g, 26.6 mmol). The reaction mixture was stirred at room temperature for 2 h. The reaction mixture was diluted with EtOAc, and washed with H₂O and brine. The organic layers was dried over Na₂SO₄. After concentration under reduced pressure, the residue was purified by column chromatography (Hexane/EtOAc = 4/1) to yield (1R,3R)-N-[3-(3-pyridyl)cyclopentyl]phthalimide (6.27 g, 89%) as colorless powder.
¹H-NMR(CDCl₃-d, 400 MHz) δ 1.66-1.82 (1H, m), 2.09 (1H, dt, J = 17.9, 6.7 Hz), 2.16-2.43 (3H, m), 2.45-2.56 (1H, m), 3.67-3.79 (1H, m), 4.87-4.99 (1H, m), 7.22-7.28 (1H, m), 7.59 (1H, d, J = 7.3 Hz), 7.69-7.76 (2H, m), 7.81-7.89 (2H, m), 8.46 (1H, dd, J = 4.9, 1.2 Hz), 8.56 (1H, d, J = 1.8 Hz).
CIMS (+) : 293.1 [M+H]⁺.

### Step 6: (1R,3R)-3-(3-pyridyl)cyclopentylamine

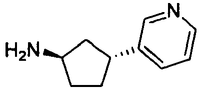

A mixture of (1R,3R)-N-[3-(3-pyridyl)cyclopentyl]phthalimide (3.00g, 10.3mmol) and hydrazinemonohydorate (1.00mL, 20.5mmol) in EtOH (40mL) was stirred at room temperture for 1 h. The reaction mixture was filtered followed by the removal of solvent. The crude product was purified by column chromatography (NH, CHCl₃ / MeOH = 50/1) to yield (1R,3R)-3-(3-pyridyl)cyclopentylamine (1.63g, 97%) as yellow oil.
¹H-NMR(CDCl₃-d, 400 MHz) δ 1.33-1.75 (4H, m), 1.81-1.95 (2H, m), 2.05-2.30 (2H, m), 3.29-3.41 (1H, m), 3.62-3.71 (1H, m), 7.21 (1H, dd, J = 7.3, 4.9 Hz), 7.52 (1H, d, J = 7.9 Hz), 8.43 (1H, dd, J = 4.9, 1.2 Hz), 8.49 (1H, d, J = 1.8 Hz).
CIMS (+) : 163.1 [M+H]⁺.

### Step 7:

### (3S)-8-amino-9-fluoro-2,3-dihydro-3-methyl-7-oxo-10-[(1S,3S)-3-(3-pyridyl)cyclope ntylamino]-7H-pyrido[1,2,3-de][1,4]benzoxazine-6-carbonitrile

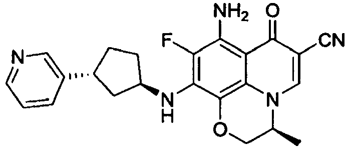

A mixture of (3S)-8-amino-9,10-difluoro-2,3-dihydro-3-methyl-7-oxo-7H-pyrido[1,2,3-de][1,4]ben zoxazine-6-carbonitrile (1.00 g, 3.61 mmol), (1R,3R)-3-(3-pyridyl)cyclopentylamine (702 mg, 4.33 mmol) and diisopropylethylamine (1.38 mL, 7.94 mmol) in anhydrous DMSO (18 mL) was stirred at 91 °C for 7 h. After cooling, the reaction mixture was poured into ice-water and then extracted with CH₂Cl₂-MeOH. The organic layer was washed with water and brine, and dried over anhyd Na₂SO₄ followed by the removal of solvent. The crude product was purified by column chromatography (EtOAc) to yield (3S)-8-amino-9-fluoro-2,3-dihydro-3-methyl-7-oxo-10-[(1S,3S)-3-(3-pyridyl)cyclope ntylamino]-7H-pyrido[1,2,3-de][1,4]benzoxazine-6-carbonitrile (247mg, 16%) as a yellow powder.
¹H-NMR(DMSO-d₆, 400 MHz) δ 1.35 (3H, d, J = 6.7 Hz), 1.54-1.80 (2H, m), 1.86-1.98 (1H, m), 2.02-2.29 (3H, m), 4.06 (1H, d, J = 9.7 Hz), 4.33 (1H, d, J = 10.3 Hz), 4.40-4.54 (2H, m), 5.23 (1H, d, J = 6.1 Hz), 6.94 (2H, brs), 7.30 (1H, dd, J = 7.9, 4.2 Hz), 7.67 (1H, d, J = 7.9 Hz), 8.38 (1H, d, J = 3.0 Hz), 8.43-8.50 (2H, m).
ESIMS (+) : 420.2 [M+H]⁺.
HRESIMS (+) : 420.18332 (Calcd for C₂₃H₂₃FN₅O₂, 420.18358).

### Example 9:

### (3S)-8-amino-9-fluoro-2,3-dihydro-3-methyl-7-oxo-10-[trans-3-(3-pyridyl)cyclohexyl amino]-7H-pyrido[1,2,3-de][1,4]benzoxazine-6-carbonitrile

### Step 1: 3-(3-pyridyl)-2-cyclohexen-1-one

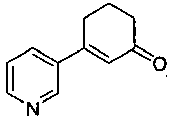

To a solution of n-BuLi (1.54 M in Hexane, 145 mL. 224 mmol) in Et₂O (600 ml) was added dropwidse 3-Bromopyridine (22.0mL, 224mmol) at -75∼-68 °C, and stirred for 20min. To a reaction mixture was added dropwise 3-ethoxy-2-cyclohexenone (25.0mL, 187mmol) at -75∼-69 °C, and stirred at room temperture for 1 h. The reaction mixture was acidified with 2M HCl (300mL), and aqueous layer was washed with AcOEt. The aqueous layer was basified with 1M NaOH aq., and then extracted with CH₂Cl₂. The organic layer was washed with brine, and dried over anhyd Na₂SO₄ followed by the removal of solvent. The crude product was purified by column chromatography (Hexane/EtOAc = 1/2) to yield 3-(3-pyridyl)-2-cyclohexen-1-one (21.5g, 66%) as yellow powder.
¹H-NMR(CDCl₃-d, 400 MHz) δ 2.15-2.26 (2H, m), 2.52 (2H, t, J = 6.7 Hz), 2.75-2.84 (2H, m), 6.43 (1H, s), 7.36 (1H, dd, J = 7.9, 4.9 Hz), 7.82 (1H, dt, J = 5.0, 2.6 Hz), 8.64 (1H, dd, J = 4.9, 1.2 Hz), 8.80 (1H, d, J = 1.8 Hz).
EIMS (+) : 173.1 [M]⁺.

### Step 2: cis-3-(3-pyridyl)cyclohexanol

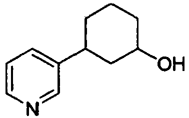

A mixture of 3-(3-pyridyl)-2-cyclohexen-1-one (19.1g, 110mmol) and 10% Pd/C (1.91g) in EtOH (300mL) was stirred under hydrogen atmosphere for 16 h. The catalyst was removed by filtration over Celite and the filtrate was concentrated in vacuo. To a solution of crude product in MeOH (360ml) was added portionwidse NaBH₄ (2.08g. 55.0mmol) at 3 °C, and stirred for 30min at same temperature. The reaction mixture was poured into ice-water and then extracted with AcOEt. The organic layer was dried over anhyd Na₂SO₄ followed by the removal of solvent. The crude product was purified by column chromatography (NH, Hexane/EtOAc = 2/1) to yield cis-3-(3-pyridyl)cyclohexanol (14.6g, 75%) as colorless oil.
¹H-NMR(CDCl₃-d, 400 MHz) δ 1.22-1.55 (4H, m), 1.79-1.99 (2H, m), 2.03-2.23 (2H, m), 2.62 (1H, tt, J = 12.4, 3.2 Hz), 3.76 (1H, t, J = 10.3 Hz), 7.23 (1H, dd, J = 7.9, 4.8 Hz), 7.52 (1H, d, J = 7.9 Hz), 8.42-8.52 (2H, m).
CIMS (+) : 178.1 [M+H]⁺.

### Step 3: trans-N-[3-(3-pyridyl)cyclohexyl]phthalimide

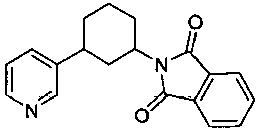

A solution of cis-3-(3-pyridyl)cyclohexanol (5.00g, 28.2mmol) in THF (100mL) was cooled to 4°C and treated with phthalimide (4.56g, 31.0mmol), Diisopropylazodicarboxylate (6.10mL, 31.0mmol) and triphenylphosphine (8.13g, 31.0mmol). The reaction mixture was stirred at room temperature for 2 h. The reaction mixture was diluted with EtOAc, and washed with H₂O and brine. The organic layers were dried over Na₂SO₄. After concentration under reduced pressure, the residue was purified by column chromatography (Hexane/EtOAc = 4/1) to yield trans-N-[3-(3-pyridyl)cyclohexyl]phthalimide (5.18g, 60%) as colorless powder.
¹H-NMR(CDCl₃-d, 400 MHz) δ 1.45-1.63 (1H, m), 1.74-1.86 (2H, m), 1.94 (1H, tt, J = 13.3, 4.2 Hz), 2.16-2.28 (2H, m), 2.36 (1H, ddd, J = 24.5, 12.4, 3.9 Hz), 2.72 (1H, td, J = 12.7, 5.0 Hz), 3.43 (1H, brs), 4.39 (1H, tt, J =11.5, 3.8 Hz), 7.29 (1H, dd, J = 7.9, 4.8 Hz), 7.67-7.88 (5H, m), 8.48 (1H, d, J = 4.2 Hz), 8.67 (1H, d, J = 1.8 Hz).
CIMS (+) : 307.1 [M+H]⁺.

### Step 4: trans-3-(3-pyridyl)cyclohexylamine

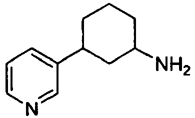

A mixture of trans-N-[3-(3-pyridyl)cyclohexyl]phthalimide (3.00g, 9.79mmol) and hydrazinemonohydorate (0.95mL, 19.6mmol) in EtOH (40 mL) was stirred at room temperture for 1.5 h. The reaction mixture was filtered followed by the removal of solvent. The crude product was purified by column chromatography (NH, EtOAc) to yield trans-3-(3-pyridyl)cyclohexylamine (1.08g, 60%) as colorless oil.
¹H-NMR(CDCl₃-d, 400 MHz) δ 1.35-1.95 (10H, m), 2.95-3.07 (1H, m), 3.38 (1H, t, J = 3.6 Hz), 7.21 (1H, dd, J = 7.9, 4.8 Hz); 7.53 (1H, d, J = 7.9 Hz), 8.43 (1H, dd, J = 4.8,1.2 Hz), 8.50 (1H, d, J = 1.8 Hz).
CIMS (+) : 177.1 [M+H]⁺.

### Step 5:

### (3S)-8-amino-9-fluoro-2,3-dihydro-3-methyl-7-oxo-10-[trans-3-(3-pyridyl)cyclohexyl amino]-7H-pyrido[1,2,3-de][1,4]benzoxazine-6-carbonitrile

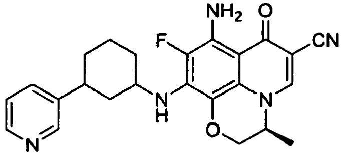

A mixture of (3S)-8-amino-9,10-difluoro-2,3-dihydro-3-methyl-7-oxo-7H-pyrido[1,2,3-de][1,4]ben zoxazine-6-carbonitrile (800 mg, 2.89 mmol), trans-3-(3-pyridyl)cyclohexylamine (612mg, 3.47mmol) and diisopropylethylamine (1.11mL, 6.36mmol) in anhydrous DMSO (12mL) was stirred at 84 °C for 9 h. After cooling, the reaction mixture was poured into ice-water and then extracted with CH₂Cl₂-MeOH. The organic layer was washed with water and brine, and dried over anhyd Na₂SO₄ followed by the removal of solvent. The crude product was purified by column chromatography (EtOAc) to yield (3S)-8-amino-9-fluoro-2,3-dihydro-3-methyl-7-oxo-10-[trans-3-(3-pyridyl)cyclohexyl amino]-7H-pyrido[1,2,3-de][1,4]benzoxazine-6-carbonitrile (309mg, 24%) as a yellow powder.
¹H-NMR(DMSO-d₆, 400 MHz) δ 1.37 (3H, dd, J = 6.7, 4.3 Hz), 1.47-1.93 (8H, m), 2.80-2.95 (1H, m), 4.06-4.22 (2H, m), 4.35-4.52 (2H, m), 4.98 (1H, d, J = 7.9 Hz), 6.95 (2H, brs), 7.29 (1H, dd, J = 7.9, 4.8 Hz), 7.64 (1H, d, J = 6.7 Hz), 8.38 (1H, dd, J = 4.8, 1.2 Hz), 8.44 (1H, s), 8.49 (1H, s).
ESIMS (+) : 434.2 [M+H]⁺.
HRESIMS (+) : 434.19998 (Calcd for C₂₄H₂₅FN₅O₂, 434.19923).

### Example 10:

### (3S)-8-amino-9-fluoro-2,3-dihydro-3-methyl-7-oxo-10-[cis-3-(3-pyridyl)cyclohexyla mino]-7H-pyrido[1,2,3-de][1,4]benzoxazine-6-carbonitrile

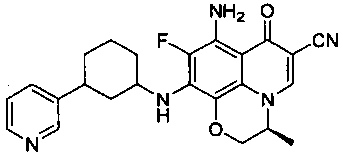

### Step 1: trans-3-(3-pyridyl)cyclohexanol

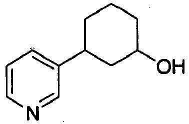

A mixture of 3-(3-pyridyl)-2-cyclohexen-1-one (19.1g, 110mmol) and 10% Pd/C (1.91g) in EtOH (300mL) was stirred under hydrogen atmosphere for 16 h. The catalyst was removed by filtration over Celite and the filtrate was concentrated in vacuo. To a solution of crude product in MeOH (360 ml) was added portionwidse NaBH₄ (2.08g. 55.0mmol) at 3 °C, and stirred for 30 min at same temperature. The reaction mixture was poured into ice-water and then extracted with AcOEt. The organic layer was dried over anhyd Na₂SO₄ followed by the removal of solvent. The crude product was purified by column chromatography (NH, Hexane/EtOAc = 2/1) to yield trans-3-(3-pyridyl)cyclohexanol (4.19 g, 21%) as colorless powder.
¹H-NMR(CDCl₃-d, 400 MHz) δ 1.38-2.10 (8H, m), 3.00-3.15 (1H, m), 4.26 (1H, brs), 7.22 (1H, dd, J = 7.9, 4.8 Hz), 7.52 (1H, d, J = 7.9 Hz), 8.40-8.53 (2H, m).
CIMS (+) : 178.1 [M+H]⁺.

### Step 2: cis-N-[3-(3-pyridyl)cyclohexyl]phthalimide

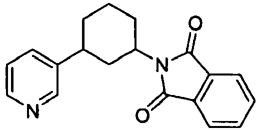

A solution of trans-3-(3-pyridyl)cyclohexanol (3.99g, 22.5mmol) in THF (90mL) was cooled to 4 °C and treated with phthalimide (3.65g, 24.8mmol), Diisopropylazodicarboxylate (4.88 mL, 24.8 mmol) and triphenylphosphine (6.50g, 24.8mmol). The reaction mixture was stirred at room temperature for 30 min. The reaction mixture was diluted with EtOAc, and washed with H₂O and brine. The organic layers were dried over Na₂SO₄. After concentration under reduced pressure, the residue was purified by column chromatography (Hexane/EtOAc = 4/1) to yield cis-N-[3-(3-pyridyl)cyclohexyl]phthalimide (1.66 g, 24%) as colorless powder.
colorless powder
¹H-NMR(CDCl₃-d, 400 MHz) δ 1.45-1.64 (2H, m), 1.79-1.98 (3H, m), 2.01-2.11 (1H, m), 2.24-2.38 (1H, m), 2.46 (1H, q, J = 12.2 Hz), 2.68-2.79 (1H, m), 4.33 (1H, tt, J = 12.2, 3.7 Hz), 7.23 (1H, dd, J = 7.3, 4.9 Hz), 7.57 (1H, d, J = 7.9 Hz), 7.67-7.75 (2H, m), 7.78-7.86 (2H, m), 8.45 (1H, dd, J = 4.9, 1.2 Hz), 8.51 (1H, d, J = 1.8 Hz).
EIMS (+) : 306.1 [M]⁺.

### Step 3: cis-3-(3-pyridyl)cyclohexylamine

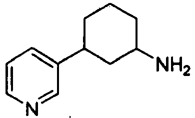

A mixture of cis-N-[3-(3-pyridyl)cyclohexyl]phtalimide (1.64 g, 5.35 mmol) and hydrazinemonohydorate (0.52 mL, 10.7 mmol) in EtOH (25 mL) was stirred at room temperture for 2 h. The reaction mixture was filtered followed by the removal of solvent. The crude product was purified by column chromatography (NH, EtOAc) to yield cis-3-(3-pyridyl)cyclohexylamine (348g, 37%) as colorless oil. ¹H-NMR(CDCl₃-d, 400 MHz) δ 1.11 (1H, ddd, J = 24.5, 12.4, 3.3 Hz), 1.20-1.70 (4H, m), 1.71-2.19 (5H, m), 2.62 (1H, tt, J = 12.1, 3.3 Hz), 2.83 (1H, tt, J =11.2, 3.8 Hz), 7.22 (1H, dd, J = 7.9, 4.8 Hz), 7.51 (1H, d, J = 7.9 Hz), 8.40-8.91 (2H, m).
EIMS (+) : 176.1 [M]⁺.

### Step 4:

### (3S)-8-amino-9-fluoro-2,3-dihydro-3-methyl-7-oxo-10-[cis-3-(3-pyridyl)cyclohexyla mino]-7H-pyrido[1,2,3-de][1,4]benzoxazine-6-carbonitrile

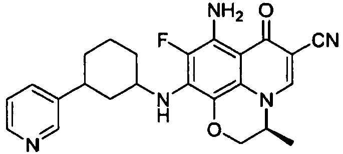

A mixture of (3S)-8-amino-9,10-difluoro-2,3-dihydro-3-methyl-7-oxo-7H-pyrido[1,2,3-de][1,4]ben zoxazine-6-carbonitrile (457 mg, 1.65 mmol), cis-3-(3-pyridyl)cyclohexylamine (348 mg, 1.97 mmol) and diisopropylethylamine (0.63 mL, 3.63 mmol) in anhydrous DMSO (8 mL) was stirred at 93 °C for 9 h. After cooling, the reaction mixture was poured into ice-water and then extracted with CH₂Cl₂-MeOH. The organic layer was washed with water and brine, and dried over anhyd Na₂SO₄ followed by the removal of solvent. The crude product was purified by column chromatography (EtOAc) to yield (3S)-8-amino-9-fluoro-2,3-dihydro-3-methyl-7-oxo-10-[cis-3-(3-pyridyl)cyclohexyla mino]-7H-pyrido[1,2,3-de][1,4]benzoxazine-6-carbonitrile (96.0mg, 13%) as a yellow powder.
¹H-NMR(DMSO-d₆, 400 MHz) δ 1.44-1.68 (8H, m), 1.81 (2H, dd, J = 37.2,11.2 Hz), 1.95-2.08 (2H, m), 2.69 (1H, t, J = 11.2 Hz), 3.75-3.88 (1H, m), 4.04 (1H, d, J = 10.9 Hz), 4.32 (1H, d, J = 11.5 Hz), 4.38-4.50 (1H, m), 4.95 (1H, d, J = 9.7 Hz), 6.94 (2H, brs), 7.30 (1H, dd, J = 7.3, 4.8 Hz), 7.60-7.68 (1H, m), 8.39 (1H, d, J = 4.2 Hz), 8.45 (2H, s).
ESIMS (+) : 434.2 [M+H]⁺.
HRESIMS (+) : 434.19890 (Calcd for C₂₄H₂₅FN₅O₂, 434.19923).

### Example 11: GS activation

HepG2 cells were obtained from the Japanese Collection of Research Bioresources and were grown in standard culture medium, a low-glucose Dulbecco's modified Eagle's medium (DMEM) containing 10% fetal calf serum supplemented with 100 U/mL penicillin and 100 µg/mL streptomycin, in a humidified and 5% CO₂ atmosphere kept at 37°C. The HepG2 cells were harvested with 0.25% trypsin solution containing 1 mM EDTA, and were seeded on 12 well plates at 1 x 10⁵ cells per well. Following a culture for 3 days, the cells were washed once with phosphate buffered saline (PBS), and were incubated with serum-free low-glucose DMEM supplemented with 100 U/mL penicillin and 100 µg/mL streptomycin. Following a culture for 3 hrs, GSK-3 inhibitor at various concentration and 2.5 µCi/mL D-[2-³ H]glucose (New England Nuclear, Boston, MA, USA) were added to the serum-free low-glucose DMEM. A vehicle control of DMSO (0.3%, final concentration) was also used. Total volume per well of the reaction medium was 1.0 mL of serum-free low-glucose DMEM. After incubation at 37°C for 3 hrs, medium was aspirated and cells were washed twice with PBS, and were added 0.25 mL of 1 N KOH containing 0.4 mg/mL carrier glycogen. After incubation at 37°C for 30 min, 0.25 mL of 48.8% (w/v) KOH was added to well for cell lysis. After incubation at 95°C for 30 min, 1.5 mL of 95% (v/v) ethanol was added to the cell lysate. Total glycogen was precipitated overnight at -20°C. Glycogen precipitates were recovered by centrifugation at 19,000 x g for 30 min at 4°C. Precipitates were washed once with 1 mL of 70% (v/v) ethanol, and were re-suspended in 0.5 mL water. [³H]Glucose incorporation into glycogen was assessed using a liquid scintillation counter (Packard Instrument Co., Meriden, CT, USA).

EC₅₀ for the glycogen synthesis of the compounds of Examples 7, 9 and 10 was 0.3µM or lower.

### Example 12: Animal Study: Oral glucose tolerance test.

Male Cr1j:CD1 (ICR) mice were obtained from Charles River Laboratories Japan (Yokohama, Japan). All mice were given a standard diet (Clea Japan, Tokyo, Japan) and tap water *ad libitum.* All institutional guidelines for animal care and use were applied in this study. Test compounds were suspended in 0.3% carboxymethyl-cellulose sodium salt (CMC-Na; Sigma, St. Louis, MO). After fasting for 15-17 hours, the test compound (3, 10, 30, 100 or 300 mg/kg) or vehicle (0.3% CMC-Na) was orally administered to 7-week-old ICR mice. Glucose solution (5g/kg) was orally administered at 30 minutes after test compound treatment. Blood samples were collected from tail vein using capillary tubes containing EDTA · K before test compound treatment, and at 0, 0.5, 1, and 2 hours after glucose load. The blood samples were centrifuged at 2,500 xg for 5 minutes and separated plasma was kept on ice and analyzed in the same day. Plasma glucose levels were determined using the glucose C II-test (Wako Pure Chemical Industries, Osaka, Japan). The sum of plasma glucose levels at 0.5 and 1 hr after glucose load was compared to that of vehicle treatment, and results were presented as percent decrease. Result for the title compound of Example 2 (10 mg/kg) was 22%.

## Claims

1. A compound of Formula (I): 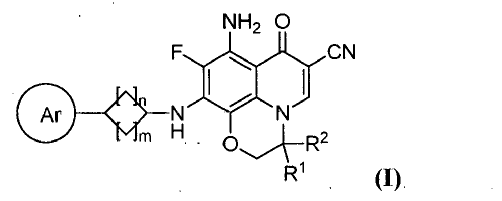 or a pharmaceutically acceptable salt thereof, wherein
R¹ is C₁₋₆ alkyl,
R² is hydrogen or C₁₋₆ alkyl;
m is 1, 2 or 3;
n is 1 or 2;
Ar is aryl or heteroaryl which is optionally substituted with one to three substituents, each independently selected from Q groups;
wherein Q is halo, hydroxy, cyano, nitro, oxo, thioxo, hydroxycarbonyl, alkyl, haloalkyl, alkenyl, alkynyl, cycloalkyl, heterocyclyl, aryl, heteroaryl, aralkyl, heteroaralkyl, alkoxy, haloalkoxy, cycloalkoxy, heterocyclyloxy, aryloxy, heteroaryloxy, aralkyloxy, heretoaralkyloxy, alkylcarbonyl, arylcarbonyl, heteroarylcarbonyl, alkoxycarbonyl, aryloxycarbonyl, aralkyloxycarbonyl, unsubstituted or substituted aminocarbonyl, alkylcarbonyloxy, arylcarbonyloxy, aralkylcarbonyloxy, alkoxycarbonyloxy, aryloxycarbonyloxy, aralkyloxycarbonyloxy, unsubstituted or substituted aminocarbonyloxy, unsubstituted or substituted amino, alkylthio, cycloalkylthio, arylthio, heteroarylthio, aralkylthio, heteroaralkylthio, alkylsulfinyl, cycloalkylsulfinyl, arylsulfinyl, heteroarylsulfinyl, aralkylsulfinyl, heteroaralkylsulfinyl, alkylsulfonyl, cycloalkylsulfonyl, arylsulfonyl, heteroarylsulfonyl, aralkylsulfonyl, heteroaralkylsulfonyl, alkoxysulfonyl, aryloxysulfonyl, unsubstituted or substituted aminosulfonyl, or hydroxysulfonyl.

2. The compound of claim 1, wherein R² is hydrogen.

3. The compound of claim 1 or 2, wherein Ar is heteroaryl which is optionally substituted with one to three substituents, each independently selected from Q groups.

4. The compound of any of claims 1-3, wherein m+n is 3 or 4.

5. The compound of claim 1, having Formula (Ia): 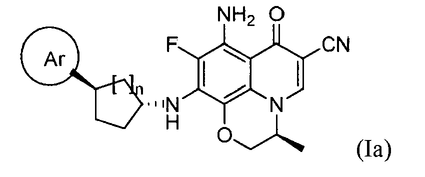 or a pharmaceutically acceptable salt thereof,
wherein n is 1 or 2;
Ar is aryl or heteroaryl which is optionally substituted with one to three substituents, each independently selected from Q groups;
wherein Q is halo, hydroxy, cyano, nitro, oxo, thioxo, hydroxycarbonyl, alkyl, haloalkyl, alkenyl, alkynyl, cycloalkyl, heterocyclyl, aryl, heteroaryl, aralkyl, heteroaralkyl, alkoxy, haloalkoxy, cycloalkoxy, heterocyclyloxy, aryloxy, heteroaryloxy, aralkyloxy, heretoaralkyloxy, alkylcarbonyl, arylcarbonyl, heteroarylcarbonyl, alkoxycarbonyl, aryloxycarbonyl, aralkyloxycarbonyl, unsubstituted or substituted aminocarbonyl, alkylcarbonyloxy, arylcarbonyloxy, aralkylcarbonyloxy, alkoxycarbonyloxy, aryloxycarbonyloxy, aralkyloxycarbonyloxy, unsubstituted or substituted aminocarbonyloxy, unsubstituted or substituted amino, alkylthio, cycloalkylthio, arylthio, heteroarylthio, aralkylthio, heteroaralkylthio, alkylsulfinyl, cycloalkylsulfinyl, arylsulfinyl, heteroarylsulfinyl, aralkylsulfinyl, heteroaralkylsulfinyl, alkylsulfonyl, cycloalkylsulfonyl, aryl sulfonyl, heteroarylsulfonyl, aralkylsulfonyl, heteroaralkylsulfonyl, alkoxysulfonyl, aryloxysulfonyl, unsubstituted or substituted aminosulfonyl,or hydroxysulfonyl.

6. The compound of claim 5, wherein Ar is heteroaryl which is optionally substituted with one to three substituents, each independently selected from Q groups.

7. The compound of claim 6, wherein Ar is pyridyl, pyramidyl, pyrazolyl or imidazolyl, which is optionally substituted with one to three substituents, each independently selected from Q groups.

8. The compound of claim 1 selected from:

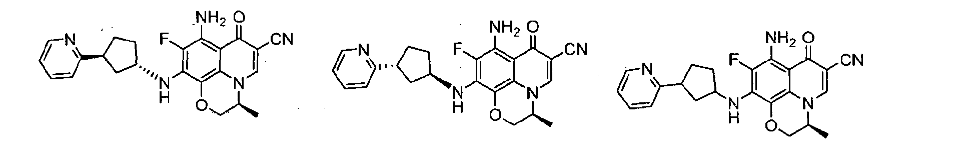

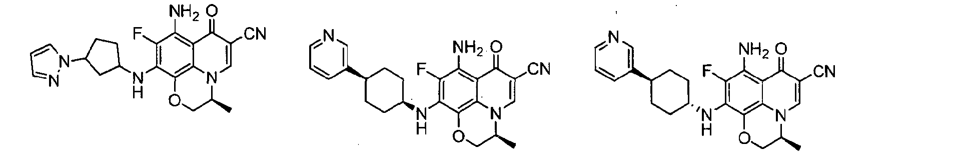

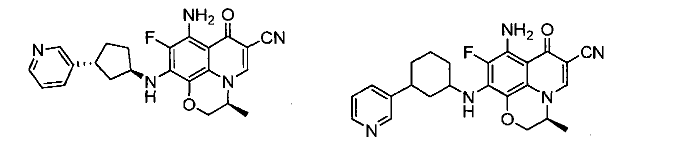

9. A pharmaceutical composition comprising a compound of any of claims 1-8 and a pharmaceutically acceptable carrier.

10. The pharmaceutical composition of claim 9 for use in treating, preventing or ameliorating GSK-3 mediated disease.

11. The pharmaceutical composition for use of claim 10, wherein said GSK-3 mediated disease is selected from the group consisting of diabetes, conditions associated with diabetes, chronic neurodegenerative conditions including dementias such as Alzheimer's disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis, neurotraumatic diseases such as acute stroke, epilepsy, mood disorders such as depression, schizophrenia and bipolar disorders, rheumatoid arthritis, inflammatory bowel disease, ulceractive colitis, Crohn's disease, sepsis, pancreatic cancer, ovarian cancer and osteoporosis.

12. The compound of any of claims 1-8 for use in treating, preventing or ameliorating a GSK-3 mediated disease.

13. The compound for use of claim 12, wherein said GSK-3 mediated disease is selected from the group consisting of diabetes, conditions associated with diabetes, chronic neurodegenerative conditions including dementias such as Alzheimer's disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis, neurotraumatic diseases such as acute stroke, epilepsy, mood disorders such as depression, schizophrenia and bipolar disorders, rheumatoid arthritis, inflammatory bowel disease, ulceractive colitis, Crohn's disease, sepsis, pancreatic cancer, ovarian cancer and osteoporosis.

## Patentansprüche

1. Eine Verbindung der Formel (I): 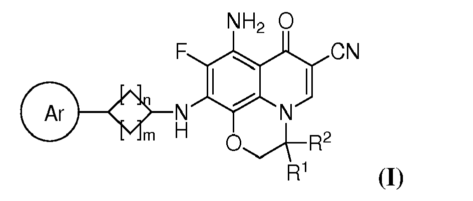 oder ein pharmazeutisch verträgliches Salz davon, wobei
R¹ C₁₋₆-Alkyl ist;
R² Wasserstoff oder C₁₋₆-Alkyl ist;
m 1, 2 oder 3 ist;
n 1 oder 2 ist;
Ar Aryl oder Heteroaryl ist, das gegebenenfalls mit einem bis drei jeweils unabhängig voneinander aus Q-Gruppen ausgewählten Substituenten substituiert ist;
wobei Q Halogen, Hydroxy, Cyano, Nitro, Oxo, Thioxo, Hydroxycarbonyl, Alkyl, Halogenalkyl, Alkenyl, Alkinyl, Cycloalkyl, Heterocyclyl, Aryl, Heteroaryl, Aralkyl, Heteroaralkyl, Alkoxy, Halogenalkoxy, Cycloalkoxy, Heterocyclyloxy, Aryloxy, Heteroaryloxy, Aralkyloxy, Heretoaralkyloxy, Alkylcarbonyl, Arylcarbonyl, Heteroarylcarbonyl, Alkoxycarbonyl, Aryloxycarbonyl, Aralkyloxycarbonyl, unsubstituiertes oder substituiertes Aminocarbonyl, Alkylcarbonyloxy, Arylcarbonyloxy, Aralkylcarbonyloxy, Alkoxycarbonyloxy, Aryloxycarbonyloxy, Aralkyloxycarbonyloxy, unsubstituiertes oder substituiertes Aminocarbonyloxy, unsubstituiertes oder substituiertes Amino, Alkylthio, Cycloalkylthio, Arylthio, Heteroarylthio, Aralkylthio, Heteroaralkylthio, Alkylsulfinyl, Cycloalkylsulfinyl, Arylsulfinyl, Heteroarylsulfinyl, Aralkylsulfinyl, Heteroaralkylsulfinyl, Alkylsulfonyl, Cycloalkylsulfonyl, Arylsulfonyl, Heteroarylsulfonyl, Aralkylsulfonyl, Heteroaralkylsulfonyl, Alkoxysulfonyl, Aryloxysulfonyl, unsubstituiertes oder substituiertes Aminosulfonyl oder Hydroxysulfonyl ist.

2. Die Verbindung nach Anspruch 1, wobei R² Wasserstoff ist.

3. Die Verbindung nach Anspruch 1 oder 2, wobei Ar Heteroaryl ist, das gegebenenfalls mit einem bis drei jeweils unabhängig voneinander aus Q-Gruppen ausgewählten Substituenten substituiert ist.

4. Die Verbindung nach einem der Ansprüche 1-3, wobei m+n 3 oder 4 ist.

5. Die Verbindung nach Anspruch 1 mit der Formel (Ia): 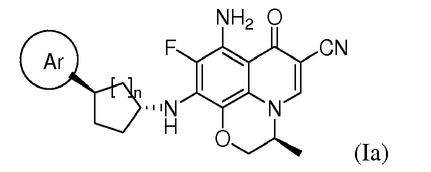 oder ein pharmazeutisch verträgliches Salz davon,
wobei n 1 oder 2 ist;
Ar Aryl oder Heteroaryl ist, das gegebenenfalls mit einem bis drei jeweils unabhängig voneinander aus Q-Gruppen ausgewählten Substituenten substituiert ist;
wobei Q Halogen, Hydroxy, Cyano, Nitro, Oxo, Thioxo, Hydroxycarbonyl, Alkyl, Halogenalkyl, Alkenyl, Alkinyl, Cycloalkyl, Heterocyclyl, Aryl, Heteroaryl, Aralkyl, Heteroaralkyl, Alkoxy, Halogenalkoxy, Cycloalkoxy, Heterocyclyloxy, Aryloxy, Heteroaryloxy, Aralkyloxy, Heretoaralkyloxy, Alkylcarbonyl, Arylcarbonyl, Heteroarylcarbonyl, Alkoxycarbonyl, Aryloxycarbonyl, Aralkyloxycarbonyl, unsubstituiertes oder substituiertes Aminocarbonyl, Alkylcarbonyloxy, Arylcarbonyloxy, Aralkylcarbonyloxy, Alkoxycarbonyloxy, Aryloxycarbonyloxy, Aralkyloxycarbonyloxy, unsubstituiertes oder substutiertes Aminocarbonyloxy, unsubstituiertes oder substituiertes Amino, Alkylthio, Cycloalkylthio, Arylthio, Heteroarylthio, Aralkylthio, Heteroaralkylthio, Alkylsulfinyl, Cycloalkylsulfinyl, Arylsulfinyl, Heteroarylsulfinyl, Aralkylsulfinyl, Heteroaralkylsulfinyl, Alkylsulfonyl, Cycloalkylsulfonyl, Arylsulfonyl, Heteroarylsulfonyl, Aralkylsulfonyl, Heteroaralkylsulfonyl, Alkoxysulfonyl, Aryloxysulfonyl, unsubstituiertes oder substituiertes Aminosulfonyl oder Hydroxysulfonyl ist.

6. Die Verbindung nach Anspruch 5, wobei Ar Heteroaryl ist, das gegebenenfalls mit einem bis drei jeweils unabhängig voneinander aus Q-Gruppen ausgewählten Substituenten substituiert ist.

7. Die Verbindung nach Anspruch 6, wobei Ar Pyridyl, Pyramidyl, Pyrazolyl oder Imidazolyl ist, das gegebenenfalls mit einem bis drei jeweils unabhängig voneinander aus Q-Gruppen ausgewählten Substituenten substituiert ist.

8. Die Verbindung nach Anspruch 1, ausgewählt aus:

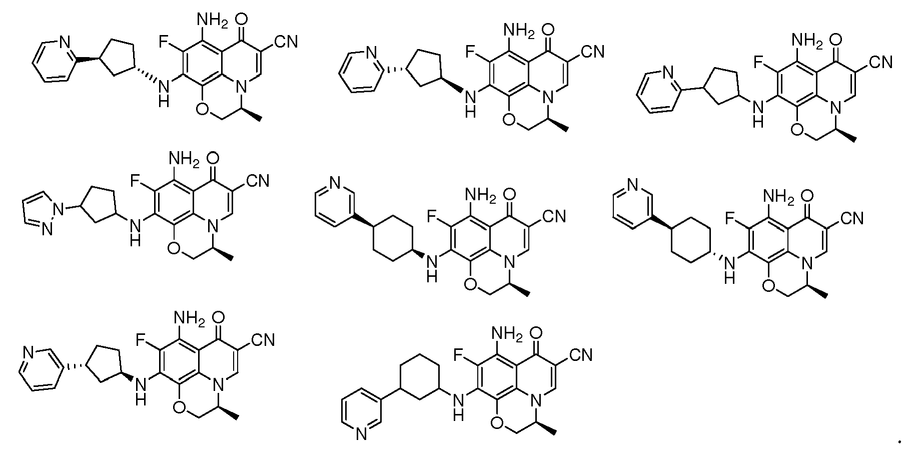

9. Eine pharmazeutische Zusammensetzung umfassend eine Verbindung nach einem der Ansprüche 1-8 und einen pharmazeutisch verträglichen Träger.

10. Die pharmazeutische Zusammensetzung nach Anspruch 9 zur Verwendung bei der Behandlung, Vorbeugung oder Linderung einer GSK-3-vermittelten Erkrankung.

11. Die pharmazeutische Zusammensetzung zur Verwendung nach Anspruch 10, wobei die GSK-3-vermittelte Erkrankung aus der Gruppe, die aus Diabetes, mit Diabetes in Zusammenhang stehenden Erkrankungen, chronischen neurodegenerativen Erkrankungen einschließlich Demenzen wie Alzheimer-Krankheit, Parkinson-Krankheit, progressiver supranukleärer Blickparese, subakuter sklerosierender panenzephalitischer Parkinson-Krankheit, postencephalitischer Parkinson-Krankheit, pugilistischer Enzephalitis, Guam-Parkinson-Demenz-Komplex, Morbus Pick, kortikobasaler Degeneration, frontotemporaler Demenz, Chorea Huntington, AIDS-assoziierter Demenz, amyotropher Lateralsklerose, Multipler Sklerose, neurotraumatischen Krankheiten wie akutem Schlaganfall, Epilepsie, affektiven Störungen wie Depression, Schizophrenie und bipolaren Störungen, rheumatoider Arthritis, entzündlicher Darmerkrankung, Colitis ulcerosa, Morbus Crohn, Sepsis, Pankreaskrebs, Eierstockkrebs und Osteoporose besteht, ausgewählt ist.

12. Die Verbindung nach einem der Ansprüche 1-8 zur Verwendung bei der Behandlung, Vorbeugung oder Linderung einer GSK-3-vermittelten Erkrankung.

13. Die Verbindung zur Verwendung nach Anspruch 12, wobei die GSK-3-vermittelte Erkrankung aus der Gruppe, die aus Diabetes, mit Diabetes in Zusammenhang stehenden Erkrankungen, chronischen neurodegenerativen Erkrankungen einschließlich Demenzen wie Alzheimer-Krankheit, Parkinson-Krankheit, progressiver supranukleärer Blickparese, subakuter sklerosierender panenzephalitischer Parkinson-Krankheit, postencephalitischer Parkinson-Krankheit, pugilistischer Enzephalitis, Guam-Parkinson-Demenz-Komplex, Morbus Pick, kortikobasaler Degeneration, frontotemporaler Demenz, Chorea Huntington, AIDS-assoziierter Demenz, amyotropher Lateralsklerose, Multipler Sklerose, neurotraumatischen Krankheiten wie akutem Schlaganfall, Epilepsie, affektiven Störungen wie Depression, Schizophrenie und bipolaren Störungen, rheumatoider Arthritis, entzündlicher Darmerkrankung, Colitis ulcerosa, Morbus Crohn, Sepsis, Pankreaskrebs, Eierstockkrebs und Osteoporose besteht, ausgewählt ist.

## Revendications

1. Composé de formule (I) : 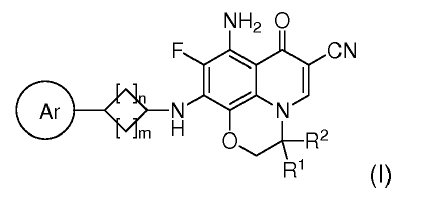 ou un sel pharmaceutiquement acceptable de celui-ci, dans lequel R¹ est un groupe alkyle en C₁₋₆ ;
R² est un atome d'hydrogène ou un groupe alkyle en C₁₋₆ ;
m vaut 1 , 2 ou 3 ;
n vaut 1 ou 2 ;
Ar est un groupe aryle ou hétéroaryle qui est éventuellement substitué par un à trois substituants, chacun indépendamment choisi parmi les groupes Q ;
dans lequel Q est un groupe halogéno, hydroxy, cyano, nitro, oxo, thioxo, hydroxycarbonyle, alkyle, halogénoalkyle, alcényle, alcynyle, cycloalkyle, hétérocyclyle, aryle, hétéroaryle, aralkyle, hétéroaralkyle, alcoxy, halogénoalcoxy, cycloalcoxy, hétérocyclyloxy, aryloxy, hétéroaryloxy, aralkyloxy, hétéroaralkyloxy, alkylcarbonyle, arylcarbonyle, hétéroarylcarbonyle, alcoxycarbonyle, aryloxycarbonyle, aralkyloxycarbonyle, aminocarbonyle non substitué ou substitué, alkylcarbonyloxy, arylcarbonyloxy, aralkylcarbonyloxy, alcoxycarbonyloxy, aryloxycarbonyloxy, aralkyloxycarbonyloxy, aminocarbonyloxy non substitué ou substitué, amino non substitué ou substitué, alkylthio, cycloalkylthio, arylthio, hétéroarylthio, aralkylthio, hétéroaralkylthio, alkylsulfinyle, cycloalkylsulfinyle, arylsulfinyle, hétéroarylsulfinyle, aralkylsulfinyle, hétéroaralkylsulfinyle, alkylsulfonyle, cycloalkylsulfonyle, arylsulfonyle, hétéroarylsulfonyle, aralkylsulfonyle, hétéroaralkylsulfonyle, alcoxysulfonyle, aryloxysulfonyle, aminosulfonyle non substitué ou substitué, ou hydroxysulfonyle.

2. Composé selon la revendication 1, dans lequel R² est un atome d'hydrogène.

3. Composé selon la revendication 1 ou 2, dans lequel Ar est un groupe hétéroaryle qui est éventuellement substitué par un à trois substituants, chacun indépendamment choisi parmi les groupes Q.

4. Composé selon l'une quelconque des revendications 1 à 3, dans lequel m+n vaut 3 ou 4.

5. Composé selon la revendication 1, ayant la formule (la) : 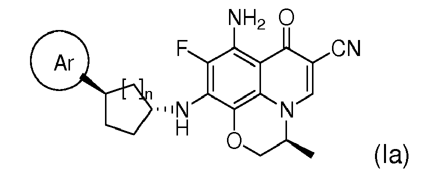 ou un sel pharmaceutiquement acceptable de celui-ci,
dans lequel n vaut 1 ou 2 ;
Ar est un groupe aryle ou hétéroaryle qui est éventuellement substitué par un à trois substituants, chacun indépendamment choisi parmi les groupes Q ;
dans lequel Q est un groupe halogéno, hydroxy, cyano, nitro, oxo, thioxo, hydroxycarbonyle, alkyle, halogénoalkyle, alcényle, alcynyle, cycloalkyle, hétérocyclyle, aryle, hétéroaryle, aralkyle, hétéroaralkyle, alcoxy, halogénoalcoxy, cycloalcoxy, hétérocyclyloxy, aryloxy, hétéroaryloxy, aralkyloxy, hétéroaralkyloxy, alkylcarbonyle, arylcarbonyle, hétéroarylcarbonyle, alcoxycarbonyle, aryloxycarbonyle, aralkyloxycarbonyle, aminocarbonyle non substitué ou substitué, alkylcarbonyloxy, arylcarbonyloxy, aralkylcarbonyloxy, alcoxycarbonyloxy, aryloxycarbonyloxy, aralkyloxycarbonyloxy, aminocarbonyloxy non substitué ou substitué, amino non substitué ou substitué, alkylthio, cycloalkylthio, arylthio, hétéroarylthio, aralkylthio, hétéroaralkylthio, alkylsulfinyle, cycloalkylsulfinyle, arylsulfinyle, hétéroarylsulfinyle, aralkylsulfinyle, hétéroaralkylsulfinyle, alkylsulfonyle, cycloalkylsulfonyle, arylsulfonyle, hétéroarylsulfonyle, aralkylsulfonyle, hétéroaralkylsulfonyle, alcoxysulfonyle, aryloxysulfonyle, aminosulfonyle non substitué ou substitué, ou hydroxysulfonyle.

6. Composé selon la revendication 5, dans lequel Ar est un groupe hétéroaryle qui est éventuellement substitué par un à trois substituants, chacun indépendamment choisi parmi les groupes Q.

7. Composé selon la revendication 6, dans lequel Ar est un groupe pyridyle, pyramidyle, pyrazolyle ou imidazolyle, qui est éventuellement substitué par un à trois substituants, chacun indépendamment choisi parmi les groupes Q.

8. Composé selon la revendication 1 choisi parmi :

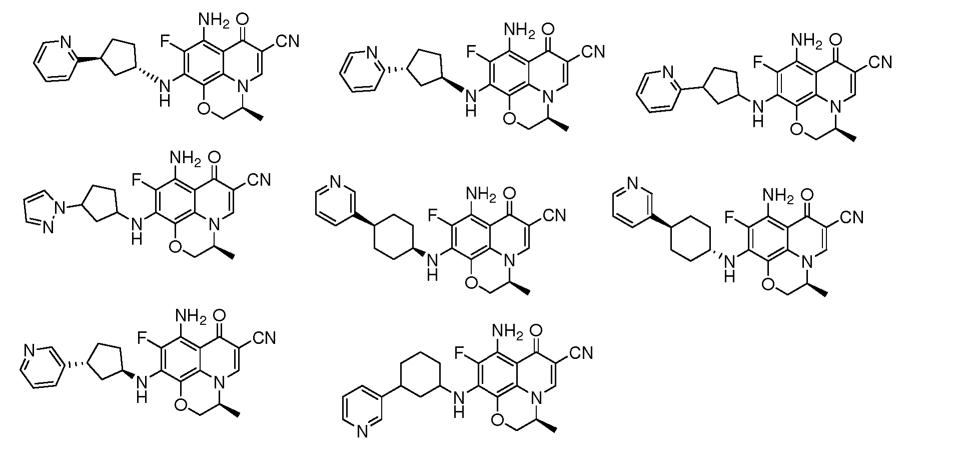

9. Composition pharmaceutique comprenant un composé selon l'une quelconque des revendications 1 à 8 et un support pharmaceutiquement acceptable.

10. Composition pharmaceutique selon la revendication 9, pour une utilisation dans le traitement, la prévention ou l'amélioration d'une maladie médiée par la GSK-3.

11. Composition pharmaceutique pour une utilisation selon la revendication 10, dans laquelle ladite maladie médiée par la GSK-3 est choisie dans le groupe constitué par le diabète, les affections associées au diabète, les affections neurodégénératives chroniques, y compris les démences comme la maladie d'Alzheimer, la maladie de Parkinson, la paralysie supranucléaire progressive, le parkinsonisme panencéphalitique sclérosant subaigu, le parkinsonisme post-encéphalitique, l'encéphalite des pugilistes, le complexe parkinsonisme-démence de Guam, la maladie de Pick, la dégénérescence cortico-basale, la démence fronto-temporale, la maladie de Huntington, la démence associée au SIDA, la sclérose latérale amyotrophique, la sclérose en plaques, les maladies neuro-traumatiques telles qu'un accident vasculaire cérébral aigu, l'épilepsie, les troubles de l'humeur tels que la dépression, la schizophrénie et les troubles bipolaires, la polyarthrite rhumatoïde, la maladie intestinale inflammatoire, la colite ulcéreuse, la maladie de Crohn, la septicémie, le cancer du pancréas, le cancer de l'ovaire et l'ostéoporose.

12. Composé selon l'une quelconque des revendications 1 à 8 pour une utilisation dans le traitement, la prévention ou l'amélioration d'une maladie médiée par la GSK-3.

13. Composé pour une utilisation selon la revendication 12, dans lequel ladite maladie médiée par la GSK-3 est choisie dans le groupe constitué par le diabète, les affections associées au diabète, les affections neurodégénératives chroniques, y compris les démences comme la maladie d'Alzheimer, la maladie de Parkinson, la paralysie supranucléaire progressive, le parkinsonisme panencéphalitique sclérosant subaigu, le parkinsonisme post-encéphalitique, l'encéphalite des pugilistes, le complexe parkinsonisme-démence de Guam, la maladie de Pick, la dégénérescence cortico-basale, la démence fronto-temporale, la maladie de Huntington, la démence associée au SIDA, la sclérose latérale amyotrophique, la sclérose en plaques, les maladies neurotraumatiques telles qu'un accident vasculaire cérébral aigu, l'épilepsie, les troubles de l'humeur tels que la dépression, la schizophrénie et les troubles bipolaires, la polyarthrite rhumatoïde, la maladie intestinale inflammatoire, la colite ulcéreuse, la maladie de Crohn, la septicémie, le cancer du pancréas, le cancer de l'ovaire et l'ostéoporose.
